# EUROPÄISCHE PATENTSCHRIFT

(11) **EP 2 516 358 B1**
(45) Veröffentlichungstag und Bekanntmachung des Hinweises auf die Patenterteilung: **23.09.2020**
(21) Anmeldenummer: 10776952.3
(22) Anmeldetag: 11.11.2010
(51) Int. Cl.: C07C 1/12, C07C 9/04

(54) **VERFAHREN ZUR HERSTELLUNG EINES METHANREICHEN PRODUKTGASES UND DAZU EINSETZBARES REAKTORSYSTEM**
METHOD FOR PRODUCING A METHANE-RICH PRODUCT GAS AND REACTOR SYSTEM USABLE FOR THAT PURPOSE
PROCÉDÉ DE PRODUCTION D'UN GAZ DE PRODUIT RICHE EN MÉTHANE, ET SYSTÈME DE RÉACTEUR UTILISABLE À CET EFFET

(30) Priorität: 23.12.2009 DE 102009059310
(43) Veröffentlichungstag der Anmeldung: 31.10.2012
(73) Patentinhaber: Hitachi Zosen Inova Etogas GmbH, 70565 Stuttgart (DE)
(72) Erfinder: ZUBERBÜHLER, Ulrich, 70563 Stuttgart (DE); STÜRMER, Bernd, 70569 Stuttgart (DE); FRICK, Volkmar, 70374 Stuttgart (DE); SPECHT, Michael, 71111 Waldenbuch (DE); BUXBAUM, Martin, A-6861 Alberschwende (AT)
(74) Vertreter: Leinweber & Zimmermann
(86) Internationale Anmeldenummer: PCT/EP2010/006877
(87) Internationale Veröffentlichungsnummer: WO 2011/076315

(56) Entgegenhaltungen:
- WO-A1-2009/007061
- MOELLER F W ET AL: "Methanation of coal gas for SNG", HYDROCARBON PROCESSING, GULF PUBLISHING CO. HOUSTON, US, Bd. 53, 1. Januar 1974 (1974-01-01), Seiten 69-74, XP008096221, ISSN: 0018-8190

## Beschreibung

Die Erfindung betrifft ein Verfahren zur Herstellung eines methanreichen Produktgases, bei dem man ein Wasserstoff und Kohlendioxid enthaltendes Eduktgas unter Einfluss wenigstens eines einstellbaren Parameters in wenigstens zwei Stufen katalytisch methanisiert und wenigstens ein die Zusammensetzung des Produktgases betreffendes Kriterium überwacht, wobei das Kriterium unter einer das Verfahren beeinflussenden Bedingung erfüllt ist, sowie ein dazu geeignetes Reaktorsystem und ein auf diese Weise hergestelltes Produktgas.

Derartige Methanisierungsverfahren sind bekannt. Sie werden beispielsweise mittels Reaktorsystemen ausgeführt, welche z.B. zwei hintereinander geschaltete Festbettreaktoren aufweisen, die mit nickelhaltigen Katalysatoren ausgestattet sind. Ein Eduktgas, das Wasserstoff und Kohlendioxid in im wesentlichen für die Methanherstellung passenden stöchiometrischen Verhältnis enthält, und darüber hinaus noch Anteile an Kohlenmonoxid, Methan, anderen Kohlenwasserstoffen sowie Verunreinigungen in Form diverser Minorkomponenten und Inertgasen (z. B. N₂) aufweisen kann, wird unter einer vorgegebenen Temperatur und einem vorgegebenen Druck dem ersten Festbettreaktor (erste Methanisierungsstufe) zugeleitet, in welchem dann die folgenden in Summe CH₄ bildenden Prozesse ablaufen:
1) CO + H₂O ↔ CO₂ + H₂ , die sogenannte water-gas shift reaction
2) CO + 3 H₂ ↔ CH₄ + H₂O, die CO-Methanisierung und
3) CO₂ + 4 H₂ ↔ CH₄ + 2 H₂O, die CO₂-Methanisierung

Auf diese Weise wird nach der ersten Methanisierungsstufe ein bestimmter Methananteil in dem aus dieser Stufe austretenden Gas erhalten. Für eine weitere Erhöhung des Methangehalts dient die nachgeschaltete zweite Methanisierungsstufe. Aus dem aus der zweiten Stufe austretenden Gas kann mit bekannten Mitteln Wasser entfernt und ggf. überschüssige Reaktanden entfernt werden, um ein Produktgas mit über 90% Methananteil zu erhalten.

Aus Gründen der sprachlichen Einfachheit wird nun und im folgenden von Gasen (Produktgas, Eduktgas,...) gesprochen, obwohl es sich natürlich um Gasgemische aus unterschiedlichen Komponenten handelt.

Das mit diesem Verfahren hergestellte methanreiche Produktgas kann bei entsprechend hohem Methangehalt als "synthetisches" Erdgas verschiedenen Bestimmungen zugeführt werden. So kann vorgesehen werden, das Produktgas in bestehende Erdgasnetze einzuspeisen. Dazu muss die DVGW Norm G 260/262 erfüllt sein, d.h. der Anteil von Wasserstoff im Produktgas muss geringer als 5% und der Anteil an Kohlenstoffdioxid geringer als 6% sein. Für die Verwendung des methanreichen Produktgases als Kraftstoff zum Antrieb von Fahrzeugen muss die DIN 51624 erfüllt sein, d.h. der Anteil von Inertgas in dem Gasgemisch muss geringer als 2% und der Anteil von Kohlendioxid geringer als 15% sein. Eine solche Verwendung des hergestellten Brenngases ist beispielsweise in der DE 10 2008 053 34 A1 offenbart.

Die zur Methanisierung eingesetzten Reaktorsysteme arbeiten üblicherweise im kontinuierlichen Betrieb, bei dem eine oder mehrere Verfahrensparameter passend zu dem fortlaufend zugeführten Eduktgas gewählt sind, um ein z.B. die oben angegebene Norm erfüllendes Produktgas zu erzeugen. Testverfahren für einen solchen, allerdings für Eduktgas aus gasifizierter Kohle ausgelegten Reaktor sind in XP 008096221 von F.W. Moeller et al offenbart.

Der Erfindung liegt die Aufgabe zugrunde, ein solches Verfahren insbesondere mit Blick auf eine erhöhte Flexibilität zu verbessern.

In verfahrenstechnischer Hinsicht wird diese Aufgabe durch eine Weiterbildung des eingangs genannten Verfahrens gemäß Anspruch 1 gelöst. Dabei nimmt man bei einer Änderung der Bedingung, hier des Eduktgasstroms, eine die Erfüllung des Kriteriums, hier des Methangehalts des Produktgases, wahrende Änderung der Einstellung des Parameters vor, hier des Drucks.

Auf diese Weise kann flexibel auf eine Änderung der Verfahrensbedingungen reagiert werden, die beispielsweise auch gewünscht herbeigeführt werden kann, wobei gleichzeitig sichergestellt wird, dass die gewünschte Produktgasqualität beibehalten wird.

Das die Zusammensetzung des Produktgases betreffende Kriterium kann ein Einzelkriterium sein, neben dem Methangehalt des Produktgases aber auch zwei oder mehr zusätzliche Subkriterien aufweisen, beispielsweise einen maximalen Wasserstoff- und/oder Kohlendioxidgehalt.

Erfindungsgemäß lässt man das Eduktgas zur ersten Methanisierungsstufe strömen und die Bedingung betrifft den Zustrom des Eduktgases. Auf diese Weise wir eine erhöhte Flexibilität hinsichtlich des Eduktgaszustroms erreicht, die die bloße zuströmende Menge pro Zeiteinheit betrifft, zusätzlich aber auch die Zusammensetzung des Eduktgases betreffen kann, auf dessen Schwankungen somit flexibel reagiert werden kann.

So ist für die pro Zeiteinheit strömende Menge des Eduktgases auch bei im Wesentlichen gleicher Zusammensetzung des Eduktgases erkannt worden, dass eine Schwankung der Menge des umzusetzenden Eduktgases erheblichen Einfluss auf das Methanisierungsverfahren nimmt, auf welchen jedoch mit der erfindungsgemäßen Lösung reagiert werden kann. Dies ist insbesondere dann von Vorteil, wenn es sich bei der Schwankung nicht um eine einmalig auftretende Störung handelt, sondern auf wiederholt auftretende Schwankungen reagiert werden soll. Insbesondere kann dann bewusst auf eine kontinuierliche Versorgung des Reaktorsystems mit einem konstanten Eduktgasstrom verzichtet werden, was die Flexibilität des Verfahrens weiter erhöht.

Hinsichtlich der Bereitstellung des Eduktgases stellt beispielsweise die Bereitstellung des CO₂-Anteils des Eduktgases keine besonderen Probleme dar, da dieser z.B. mittels Gaswäsche aus Luft erhalten werden und somit als reines CO₂ zugeführt werden kann, oder auch mit einem geringen Anteil von CH₄-Schlupf aus Biogasaufbereitung, oder alternativ in Form eines Rohbiogases, das z.B. einen ca. 45% CO₂-Anteil bei bereits ca. 55% Methananteil aufweist (nebst hier vernachlässigten Minorkomponenten). Die Bereitstellung des für die Methanisierung erforderlichen Wasserstoffs im richtigen stöchiometrischen Verhältnis ist dagegen aufwendiger, da H₂ erst unter Energieaufwand hergestellt werden muss. Dies kann beispielsweise dadurch geschehen, dass der Wasserstoff an Ort und Stelle über Elektrolyseure erzeugt wird. Dabei stellt der Elektrolyseur selbst eine an ein elektrisches Versorgernetz angeschlossene Last dar, wobei dessen maximaler Umsatz eine entsprechende Maximallast definiert. Der elektrolytisch hergestellte Wasserstoff wird vor der ersten Methanisierungsstufe mit dem Kohlendioxid enthaltenden Beitrag zum Eduktgas vermischt, so dass die aktuelle Last des Elektrolyseurs auch im wesentlichen die pro Zeiteinheit umzusetzende Menge des Eduktgases vorgibt.

Auf diese Weise kann das Methanisierungsverfahren zur Speicherung von über die Zeit nicht kontinuierlich erzeugter regenerativer Energien genutzt werden, beispielsweise Wind- oder Sonnenenergie. Der Elektrolyseur nimmt bei geringer regenerativer Energieerzeugung und fehlendem Ausgleich durch andere zugeschaltete Energiequellen eine entsprechend geringere Last ab, die für Lastwechsel, auch schnelle Lastwechsel sorgt, auf die mit dem erfindungsgemäßen Verfahren reagiert werden kann.

Das Verfahren ist somit für einen intermittierenden Betrieb zwischen dem Normalzustand und einer niedrigeren Auslastung von auch 60% oder weniger, bevorzugt 40% oder weniger, insbesondere auch 25% oder weniger der maximalen Auslastung ausgelegt. Zwischen diesen Zuständen niedriger Last und dem Normalzustand kann zur Leistungssteigerung in Umschaltzeiten von weniger als 10 Minuten, bevorzugt weniger als 4 Minuten, insbesondere weniger als einer Minute geschaltet werden, die Leistungsreduktion ist in den jeweils halben Zeiten möglich.

In einer besonders bevorzugten Ausführungsform wirkt der Einfluss des wenigstens einen Parameters zwischen der ersten und der zweiten der wenigstens zwei Methanisierungsstufen. Auf diese Weise kann der modulare Aufbau der Verfahrensstufen vorteilhaft für die Steuerung des Verfahrens bei gewahrtem erfüllten Kriterium genutzt werden, insbesondere wird dadurch die energieoptimierte Verfahrenssteuerung vereinfacht. Der erfindungsgemäß jedenfalls herangezogene Parameter ist der Druck, der demnach insbesondere zwischen der ersten und der zweiten Methanisierungsstufe geändert werden kann.

In einer Grundsteinstellung bei stationärer (Voll-)Last kann ein Gasdruck bei Eintritt in die erste Stufe 1 bar oder mehr und Stufe von 16 bar oder weniger, bevorzugt 8 bar oder weniger, insbesondere 4 bar oder weniger eingestellt werden, vor dem zweiten Reaktor ein Gasdruck zwischen 1 und 16 bar, bevorzugt zwischen 3 und 10 bar, insbesondere zwischen 5 und 8 bar. Dabei handelt es sich hier und auch im Folgenden um Absolutdrücke.

Ein weiterer Parameter, dessen Einfluss zwischen den Methanisierungsstufen wirkt, ist der Wasseranteil im Gas nach der ersten Stufe. Diesbezüglich sieht die Erfindung eine teilweise Wasserentnahme als vorteilhaft an.

Im Rahmen der Erfindung wird auch eine Kombination dieser beiden Aspekte, die erfindungsgemäße Druckänderung sowie eine Taupunkteinstellung zwischen den Stufen als vorteilhaft angesehen.

In diesem Zusammenhang ist eine weitere Spezifizierung bevorzugt, bei der das teilweise Entfernen des Wassers unter Abkühlen des Zwischengasgemisches auf eine Temperatur erreicht wird, welche unterhalb des Taupunktes liegt, aber wenigstens 60°C, bevorzugt wenigstens 80°C, insbesondere wenigstens 86°C und nochmals bevorzugt wenigstens 100°C beträgt, und/oder eine Spezifizierung, bei der die Abkühltemperatur des Zwischengasgemisches 160°C oder kleiner ist, bevorzugt 135°C oder kleiner, insbesondere 128°C oder kleiner. Insbesondere ist diesbezüglich auch vorgesehen, dass der Wassergehalt des weiterströmenden Zwischengasgemisches in Mol-Prozent 20% oder größer, bevorzugt 25% oder größer, insbesondere 30% oder größer und/oder 70% oder kleiner, bevorzugt 50% oder kleiner, insbesondere 35% oder kleiner ist. Vorzugsweise wird feuchtes Gas verdichtet. Ist ein dazu vorgesehener Kompressor nicht in der Lage, ist eine vollständige Kondensation zu wählen. Der Wassergehalt bei Eintritt in die erste Stufe ist vom CₓH_{y} Gehalt bei Reaktoreintritt abhängig und kann 0-50 mol%, bevorzugt 0-30 mol% betragen.

Bei voller Auslastung des das Verfahren betreibenden Reaktorsystems, der im Folgenden auch als Normalzustand bezeichnet wird, soll das Verfahren bezüglich weiterer Parameter bevorzugt mit bestimmten Grundeinstellungen durchgeführt werden, welche im Folgenden angegeben sind.

### Angestrebte Temperaturen:

Das Eduktgas soll mit einer Temperatur im Bereich von 250°C bis 330°C, bevorzugt 270°C bis 290°C in die erste Methanisierungsstufe eingeleitet werden. Dazu kann das Eduktgas vorgewärmt werden, insbesondere unter Einsatz der aufgrund der exothermen Methanisierung gewonnenen Abwärme. Dies verhindert das Auskühlen des Eintrittsbereichs der Stufe und die Bildung einer dadurch bedingten inaktiven Zone, sowie bei Verwendung eines Ni-haltigen Katalysators die Gefahr einer Nickelcarbonylbildung.

Im ersten Reaktor sollten Maximaltemperaturen im Bereich von 350°C bis 650°C, bevorzugt 480°C bis 580°C auftreten, wobei alternativ ein in Strömungsrichtung des Gases negativer Temperaturgradient durch entsprechende Kühlkreisführung vorgesehen werden kann, oder bevorzugt im Gleichstrom gekühlt wird. Die Austrittstemperatur kann im Bereich 280°C bis 400°C liegen, bevorzugt 300°C bis 350°C.

In der zweiten Methanisierungsstufe sollten dagegen geringere Maximaltemperaturen im Bereich von 280°C bis 400°C, bevorzugt 260°C bis 280°C herrschen, bei Eintrittstemperaturen im Bereich von 250°C bis 350°C, bevorzugt 270°C bis 330°C und Austrittstemperaturen im Bereich von 230°C bis 350°C, bevorzugt 270°C bis 300°C.

Für die Erzeugung von negativen Temperaturgradienten in den Reaktoren wird ein zur Kühlung des Gases eingesetzter Kreislauf in Gegenstromrichtung zum Gasstrom gerichtet, bevorzugt wird aber eine Kühlung im Gleichstrom eingesetzt.

### Angestrebte Raumgeschwindigkeiten:

Diese liegen in der ersten Methanisierungsstufe in einem Bereich von 2000/h bis 12000/h, bevorzugt in einem Bereich von 2000/h bis 8000/h. In der zweiten Methanisierungsstufe werden Raumgeschwindigkeiten in einem Bereich von 1000/h bis 6000/h, bevorzugt in einem Bereich von 1500/h bis 4000/h angestrebt. Bei niedriger Auslastung des Reaktorsystems sind die Raumgeschwindigkeiten entsprechend herabgesetzt. Alle Raumgeschwindigkeiten hier und im folgenden sind auf trockene Gasströme bezogen.

### Angestrebte Rezirkulation:

Das Verhältnis aus rezirkulierter Gasmenge zur Eduktgasmenge liegt bevorzugt im Bereich von 0 bis 5, insbesondere im Bereich von 0 bis 1,5 bei einem im Wesentlichen nur aus CO₂ und H₂ bestehenden Eduktgas, und 0 bis 0,5 bei Verwendung eines Biogases als CO₂ enthaltende Komponente des Eduktgases.

### Angestrebte Methanisierunasraten:

Nach Austritt aus der ersten Stufe wird ein Anteil Konvertierung von CO₂ in CH₄ von 50 bis 90vol%, bevorzugt 70 bis 80vol% angestrebt, nach der zweiten Stufe von wenigstens 75vol%, bevorzugt wenigstens 90vol%, insbesondere wenigstens 93vol% oder auch wenigstens 95vol%.

Der wenigstens eine Parameter umfasst den bei Eintritt in die erste Methanisierungsstufe herrschenden Druck und/oder den bei Eintritt in die zweite Methanisierungsstufe herrschenden Druck und als Gegensteuerung die Menge eines abgezweigten und vor der ersten Stufe rezirkulierten Gases sowie ggf. weitere Parameter aus einer Gruppe gewählt, die aufweist: den Wassergehalt des Gases vor der ersten Methanisierungsstufe; den Wassergehalt des Gases vor der zweiten Methanisierungsstufe; eine nach der ersten Methanisierungsstufe aus dem Gasgemisch entfernte Wassermenge; eine dem Erwärmen des Gases vor der ersten und/oder zweiten Methanisierungsstufe dienende Heizleistung; eine Einbeziehung JA/NEIN einer dritten Methanisierungsstufe; die Temperatur in der ersten oder (separat) in der zweiten Methanisierungsstufe; die Zusammensetzung des Eduktgases.

Von diesen Parametern wurden einige bereits oben erläutert. Als insbesondere im Hinblick auf die energetische Optimierung berücksichtigbarer weiterer Parameter ist die Menge des vor den jeweiligen Stufen zugeführten Wassers, sofern erforderlich, anzusehen. Die mögliche Einbeziehung einer dritten Methanisierungsstufe erhöht die Flexibilität des Verfahrens weiter und nutzt den erfindungsgemäß vorgesehenen modularen Aufbau des dem Verfahren zugrundeliegenden Reaktorkonzepts.

In einer besonders bevorzugten Ausführungsform erfolgt die Einstellung der Änderungen automatisch. So kann z.B. vorgesehen werden, das Verfahren in einem Energiesparmodus ablaufen zu lassen, in dem der wenigstens eine Parameter, bevorzugt zwei oder mehr Parameter automatisch energiesenkend eingestellt werden. Der Energiesparmodus kann bevorzugt durch ein Signal ausgelöst werden, dass die Änderung von der ersten auf die zweite Bedingung vollzogen ist, mit anderen Worten, dass die berücksichtigte Verfahrensbedingung wieder als stationär angesehen wird. Ein diesbezügliches Prüfkriterium kann so gestaltet sein, dass ein Signal ausgelöst wird, sobald sich die betrachtete Verfahrensbedingung über einen vorgegebenen Zeitraum nicht über ein vorgegebenes Maß hinaus ändert. Alternativ kann auch vorgesehen werden, das Verfahren grundsätzlich im Energiesparmodus laufen zu lassen, und dieses nur auf ein vorgegebenes Unterbrechungssignal zu unterbrechen, das beispielsweise ausgelöst wird, wenn das Verfahren von einer niedrigen Auslastung zu einer höheren Auslastung gebracht werden soll. Dies ist insbesondere beim oben beschriebenen intermittierenden Betrieb von Bedeutung.

In diesem Zusammenhang kann die automatische Einstellungsänderung über eine Regelung realisiert werden. Bevorzugt wird dazu ein erster Regelkreis zur Regelung der Druckänderung zwischen der ersten und der zweiten Stufe vorgesehen. Alternativ, bevorzugt zusätzlich ist ein zweiter Regelkreis zur Regelung der rezirkulierten Gasmenge vorgesehen. Desweiteren sieht die Erfindung einen dritten und vierten Regelkreis zur Regelung des Wassergehalts im Gas vor der ersten/zweiten Methanisierungsstufe vor. Über zwei oder mehr ineinander eingreifende Regelkreise kann die energetisch optimierte Verfahrenssteuerung weiter verbessert werden.

In manchen Fällen kann es zweckmäßig sein, wenn die Einstellungsänderung einer einzuhaltenden Randbedingung unterliegt. Als Beispiele für Randbedingungen können verwendet werden: Maximaltemperatur des Gases oder des Katalysators in der ersten und/oder zweiten Methanisierungsstufe; Mindest- und/oder Maximalwassergehalt des Gases vor der ersten und/oder zweiten Methanisierungsstufe; Mindest- und/oder Maximalmenge des rezirkulierten Gases; Mindest- und/oder Maximalmenge des nach dem ersten Methanisierungsschritt entfernten Wassers; Mindest- und/oder Maximalwert des in der ersten Methanisierungsstufe herrschenden Drucks; Erfüllung eines weiteren (zweiten) die Zusammensetzung des hergestellten Gasgemisches betreffenden Kriteriums.

Beispielsweise kann die Einstellung des Drucks vor der ersten Methanisierungsstufe mit der Sollmaßgabe geregelt werden, dass das Kriterium erfüllt wird, wobei der Regelung als einzuhaltende Randbedingung aufgetragen wird, eine Maximaltemperatur des Gases in der ersten Methanisierungsstufe nicht zu überschreiten. Somit kann die Randbedingung auch als zweites Regelkriterium in eine Regelung eingebunden werden. Eine weitere vorteilhafte Anwendung ist die Regelung der Wasserentnahme nach der ersten Methanisierungsstufe, die den Wassergehalt vor der zweiten Methanisierungsstufe (mit)regelt, und der Randbedingung unterliegen kann, dass ein nachgeschalteter Verdichter zur Regelung des Drucks in der zweiten Methanisierungsstufe für einen solchen Wassergehalt betreibbar ist.

Die Steuerungen/Regelungen von mehr als einem Parameter können erfindungsgemäß auch ineinandergreifen. Dazu können die Regelungen parallel und gleichzeitig ablaufen, es kann aber auch vorgesehen werden, eine erste Regelung in Abhängigkeit einer zweiten Regelung durchzuführen und letztere für unterschiedliche Sollparameter zu variieren, wonach eine energieoptimierte Regelung des ersten Parameters für jede Variation durchgeführt und deren Ergebnis verglichen wird, wonach für die zweite Regelung derjenige Sollwert ausgewählt wird, bei welchem für den ersten Parameter das absolute Energieminimum erreichbar ist. Eine solche Variante kann insbesondere dann Anwendung finden, wenn die Sollgröße ohnehin nur empirisch oder durch geeignete Modelle vorgebbar ist.

Es ist vorgesehen, die Einstellungsänderung des wenigstens einen Parameters im Falle einer Erhöhung der pro Zeiteinheit strömenden Menge des Eduktgases in einem ersten Eingriff derart vorzunehmen, dass die Reaktionsgeschwindigkeit erhöht wird. Dazu wird der Druck erhöht und die Menge des rezirkulierten Gases angepasst. Dieser Eingriff kommt besonders dann vorteilhaft zur Anwendung, wenn bei einem beabsichtigten Hochfahren die Energieoptimierung hinter einer schnellen Erhöhung der Reaktorleistung zurücktritt. In diesem Fall kann der oben erläuterte Energiesparmodus abgeschaltet werden, und das Verfahren wird in einen "Hochfahr"-Modus versetzt.

Zudem wird in einem zweiten Eingriff eine Gegensteuerung vorgenommen, sobald eine (negative) Randbedingung erfüllt wird, insbesondere das Überschreiten einer vorgegebenen Temperatur in der ersten Methanisierungsstufe erreicht wird. Auf diese Weise wird ein wirksamer Schutz für die einzelnen Komponenten, insbesondere die Katalysatoren des mit dem Verfahren betriebenen Reaktorsystems vor eine schnellere Hochschaltung zu höheren Leistungen gestellt. Die Gegensteuerung besteht jedenfalls in der Erhöhung der Menge des rezirkulierten Gases, ggf. zusätzlich wenigstens teilweise in einer Erhöhung der Reaktorkühlleistung.

Zweckmäßig wird dann in einem dritten Eingriff eine energiesparende Steuerung nach einem der vorhergehend beschriebenen Aspekte vorgenommen, sobald bezüglich der geänderten Bedingung ein stationärer Zustand erreicht ist. Hierbei werden die im ersten und zweiten Verfahrensschritt ohne Berücksichtigung der Energiebilanz vorgenommenen Einstellungen wieder korrigiert. Das Verfahren wird in den Energiespar-Modus geschaltet.

Die sich ändernde Bedingung kann zusätzlich auch noch die Zusammensetzung des Eduktgasstromes betreffen, insbesondere den Kohlendioxidanteil desselben. Somit kann eine gleichbleibende Produktqualität auch bei schwankender Eduktgaszusammensetzung erreicht werden.

Zusätzlich kann die Bedingung zweckmäßig auch die Abnutzung wenigstens eines der in den Methanisierungsstufen verwendeten Katalysatoren betreffen. Somit kann einem die Produktgasqualität vermindernden Einfluss aufgrund der schleichenden, unvermeidbar nachlassenden Aktivität der Katalysatoren entgegengewirkt werden.

Die rasche Reaktion auf geänderte Bedingungen mit den oben erläuterten geringen Anpassungszeiten kann auch geänderte Anforderungen an das zu überwachende Kriterium selbst betreffen, beispielsweise wenn von der Erzeugung eines in ein Gasnetz einzuspeisenden Produktgases umgestellt werden soll auf ein als Antriebsgas für Fahrzeuge einzusetzenden Brenngas. In diesem Fall kann die geänderte Bedingung zusätzlich noch in der Einführung eines weiteren Subkriteriums liegen.

In vorrichtungstechnischer Hinsicht stellt die Erfindung ein Reaktorsystem zur Herstellung eines methanreichen Produktgases aus einem Wasserstoff und Kohlendioxid aufweisenden Eduktgas bereit, mit wenigstens zwei hintereinandergeschalteten, einen Katalysator aufweisenden Reaktorstufen, einer Überwachungseinrichtung zur Überwachung eines die Zusammensetzung des Produktgases betreffenden Kriteriums, einer an die Überwachungseinrichtung gekoppelten Steuereinrichtung, und wenigstens einer von der Steuereinrichtung gesteuerten Einstelleinrichtung zur Einstellung wenigstens eines die Methanisierung beeinflussenden Parameters, wobei das Reaktorsystem unter einer ersten, die Methanisierung beeinflussenden Bedingung betreibbar ist und das Kriterium bei diesem Betrieb erfüllt ist, das im Wesentlichen dadurch gekennzeichnet ist, dass das Reaktorsystem dazu ausgelegt ist, unter einer Änderung der ersten Bedingung auf eine zweite Bedingung eine die Erfüllung des Kriteriums wahrende Änderung des eingestellten Parameters zu erlauben, mit den weiteren Merkmalen des Anspruchs 10, also ein Verfahren gemäß Anspruch 1 bewirkend.

Die Vorteile eines solchen Reaktorsystems ergeben sich aus den obigen Erläuterungen des Verfahrens.

Bevorzugt können sich die wenigstens zwei Reaktorstufen hinsichtlich ihres Aufbaus unterscheiden. Auf diese Weise wird zum einen ein modulartiger Reaktoraufbau erreicht, dessen einzelne Stufen dennoch individuell auf das wesentliche Anwendungsgebiet auslegbar sind. Insbesondere kann sich der Aufbau in der mechanischen Bauform, der Art einer Wärmeableitung und/oder der Art des verwendeten Katalysators unterscheiden.

Neben dem Unterschied im Aufbau können die einzelnen Reaktorstufen auch mit unterschiedlichen Verfahrensparametern betrieben werden. Dazu ist vorgesehen, dass eine Eigenschaft des einer jeweiligen Reaktorstufe zugeführten Gases insbesondere über die wenigstens eine Einstelleinrichtung veränderbar ist.

Weiter ist zweckmäßig vorgesehen, dass wenigstens eine erste Einstelleinrichtung zwischen den beiden Reaktorstufen angeordnet ist, und insbesondere einen Verdichter aufweist oder daraus besteht. Die mit der variablen Einstellbarkeit des Drucks an dieser Stelle erreichten Vorteile ergeben sich aus der obigen Erläuterung des Verfahrens. Insofern offenbart die Erfindung auch in unabhängiger Art und Weise ein Reaktorsystem mit einer ersten Reaktorstufe, einer hinter die erste Reaktorstufe geschalteten zweiten Reaktorstufe und einer Einrichtung zur Veränderung des Drucks des aus der ersten Reaktorstufe austretenden und in die zweite Reaktorstufe eintretenden Gases, die zwischen der ersten und der zweiten Reaktorstufe angeordnet ist.

Bevorzugt ist der Verdichter so ausgelegt, dass er für einen in Dampfform vorliegenden Wassergehalt des zu verdichtenden Gases bis 35%, bevorzugt bis 50%, insbesondere bis 60% betreibbar ist. Dies schafft die Möglichkeit, eine Wassergehaltsregelung vor der zweiten Stufe allein über die Wasserentnahme zwischen den beiden Reaktorstufen zu steuern/regeln.

Eine solche Einrichtung zum Entfernen wenigstens eines Teils des nach der ersten Reaktorstufe im Gas enthaltenen Wassers ist beispielsweise als eine zweite Einstelleinrichtung des Reaktorsystems vorgesehen und insbesondere dem Verdichter vorgelagert.

Dabei erlaubt die zweite Einstelleinrichtung eine beliebige Taupunkteinstellung, insbesondere wird sie derart gesteuert, dass der Wassergehalt des nach der Teil-Wasserentnahme im Gas verbleibenden Teils zwischen 0% und 50%, bevorzugt zwischen 20% bis 50%, insbesondere zwischen 25% und 35% liegen kann.

Desweiteren weist das Reaktorsystem bevorzugt eine zweite Einstelleinrichtung zum Zuführen von Wasser vor eine oder mehrere Reaktorstufen auf. Die dritte Einstelleinrichtung kann in eine entsprechende Anzahl von den einzelnen Reaktorstufen zugeordneten Untereinrichtungen aufgeteilt sein.

Desweiteren ist zweckmäßig eine vierte Einstelleinrichtung zum Erwärmen des in eine oder mehrere Reaktorstufen eingeleiteten Gases vorgesehen, die insbesondere ebenfalls aus mehreren, den einzelnen Reaktorstufen zugeordneten Untereinrichtungen bestehen kann. Dabei dient die der ersten Reaktorstufe vorgelagerte Untereinrichtung dazu, das Eduktgas zunächst auf eine die Reaktionseinleitung erlaubende Temperatur zu erwärmen. Das aus der ersten Reaktorstufe austretende Gas hatte in der Regel eine ausreichende Temperatur für die zweite Reaktorstufe, aufgrund der zur Wasserausscheidung mittels Kondensation zwischen den Reaktorstufen erfolgten Abkühlung wird die der zweiten Reaktorstufe zugeordnete Erwärmungseinrichtung jedoch benötigt, um die Temperatur des Gases wieder auf die in der zweiten Reaktorstufe gewünschte Temperatur zu bringen. Beide Erwärmungsuntereinrichtungen in Form von beispielsweise Wärmetauschern erhalten das zur Erwärmung ihres Wärmeträgers erforderliche Energie bevorzugt aus der bei der exothermen Reaktion entstehenden und aus den Reaktorstufen abgeleiteten Abwärme.

Besonders bevorzugt ist als eine weitere Einstelleinrichtung eine Rezirkulationseinrichtung zur Rückführung eines Teils insbesondere des aus der zweiten Reaktorstufe austretenden Gases an eine vor der ersten Reaktorstufe gelegene Stelle vorgesehen. Der Einsatz und die daraus erwachsenden Vorteile der Rezirkulationseinrichtung wurden bereits mit Bezug auf das Methanisierungsverfahren beschrieben, einschließlich der in der Rezirkulation einsetzbaren steuerbaren Drosselung/Verdichter.

In einer weiteren möglichen Ausführungsform weist das Reaktorsystem eine über die Steuereinrichtung zuschaltbare dritte Reaktorstufe auf. Diese Gestaltung erhöht die mit dem modularen Aufbau des Systems erreichte Flexibilität. Diesbezüglich sieht die Erfindung auch die von der genauen Steuerung unabhängige Realisierung eines Reaktorsystems mit zwei hintereinandergeschalteten, vom Gas durchlaufenen Reaktorstufen und einer optional zuschaltbaren dritten Reaktorstufe vor.

Soweit bisher beschrieben, geht das Reaktorsystem vom Erhalt eines über eine entsprechend ausgelegte Leitung zugeführten Eduktgases aus. Das System kann jedoch eine angekoppelte Mischeinrichtung zum Mischen einzelner Komponenten des Eduktgases enthalten, und mit einer entsprechenden Anzahl von Zuführleitungen versehen sein. Eine Zuführleitung kann dabei an eine Einrichtung zur Herstellung von Wasserstoff mittels elektrischer Energie ankoppelbar und insbesondere angekoppelt sein, beispielsweise einen oder mehrere der oben bereits erläuterten Elektrolyseure.

Hinsichtlich der Kohlendioxidkomponente des Eduktgases kann vorgesehen sein, dass eine ortsnah zu den Reaktorstufen angeordnete Einrichtung zur Gaswäsche ankoppelbar und insbesondere angekoppelt ist, welche aus Luft Kohlendioxid ausfiltern und der Mischeinrichtung zuführen kann. Das Kohlendioxid kann auch aus einem Biogas abgetrennt werden, wobei bis ca. 5% Methangehalt als Rest des Biogases im Eduktgas verbleiben kann.

Des Weiteren kann eine für einen steuerbaren Zustrom eines Biogases ausgelegte weitere Leitung zur Mischeinrichtung gelegt sein, über die ein vorgebbarer Anteil eines Biogases als Bestandteil des Eduktgases zuleitbar ist.

Bevorzugt sind eine oder mehrere der Einstelleinrichtungen über einen Regelkreis steuerbar. Dazu kann die Steuereinrichtung von Messsensoren Signalinformationen mit den für die Regelung erforderlichen Istwerten erhalten und eine der Regelung entsprechende Steuerinformation an die jeweilige Einstelleinrichtung übermitteln. Die bevorzugt geregelten Größen und entsprechende Einstelleinrichtungen ergeben sich aus den obigen Erläuterungen zu den Verfahrensaspekten. So ist die Steuereinrichtung insbesondere auch zur Durchführung eines Verfahrens nach einem oder mehrerer der erläuterten Verfahrensaspekte betreibbar.

Das Reaktorsystem ist erfindungsgemäß für einen intermittierenden Betrieb zwischen zwei unterschiedlich großen umzusetzenden Gasmengen ausgelegt. Erfindungsgemäß betreffen die beiden Bedingungen unterschiedliche Mengen des pro Zeiteinheit umgesetzten Eduktgases, mit anderen Worten unterschiedliche Auslastungen des Systems, insbesondere bedingt über Lastwechsel des angekoppelten Elektrolyseurs.

Im Hinblick auf eine unabhängige Realisierung der teilweisen Wasserentnahme zwischen den beiden Methanisierungsstufen ist bevorzugt vorgesehen, dass die beiden Orte zwei hintereinander angeordnete Reaktorstufen eines Reaktorsystems sind, von denen eine jede einen Katalysator enthält und in denen der jeweilige Methanisierungsschritt stattfindet, wobei das katalytische Methanisieren des in die erste Reaktorstufe eingeleiteten Eingangsgasgemisches bei Temperaturen insbesondere im Bereich von 300°C bis 600°C erfolgt, insbesondere bei dem das weiterströmende Zwischengasgemisch in die zweite Reaktorstufe eingeleitet wird und der zweite Methanisierungsschritt dort bei Temperaturen im Bereich von insbesondere 250°C bis 350°C, insbesondere bis 300°C erfolgt, wobei das Eingangsgasgemisch insbesondere einen Kohlenmonoxidgehalt von weniger als 0,1% besitzen kann.

Weitere Einzelheiten, Merkmale und Vorteile der Erfindung ergeben sich aus der nachfolgenden Beschreibung einzelner Ausführungsbeispiele unter Bezugnahme auf die beiliegenden Figuren, von denen
- Fig. 1: eine schematische Darstellung eines erfindungsgemäßen Reaktorkonzepts zeigt, und
- Fig. 2.: eine schematische Darstellung einer weiteren Ausführungsform zeigt.

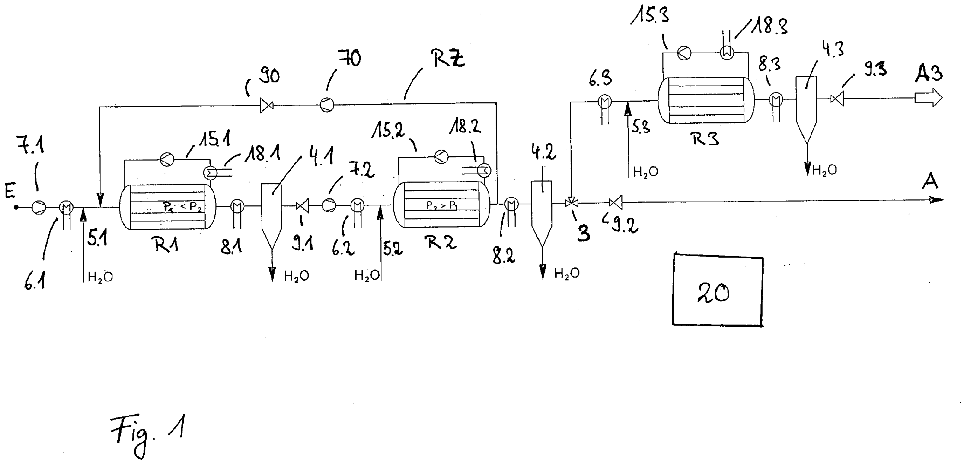

Wie aus der Darstellung von Fig. 1 hervorgeht, soll ein Eduktgas an der mit E bezeichneten Stelle dem Reaktorsystem zugeführt werden, und im Falle nur der Verwendung der Reaktoren R1 und R2 an der mit A bezeichneten Stelle der weiteren Bearbeitung übergeben werden, beispielsweise einer Einspeisung ins bestehende Erdgasnetz oder einer Bereitstellung als Brenngas für Fahrzeuge aller Art.

Auf dem Weg zwischen den Stellen E und A durchläuft das Eduktgas zwei Reaktoren, den Reaktor R1 und den nachgeschalteten Reaktor R2, die jeweils mit einem Katalysator ausgestattet sind und ein Wärmeableitungssystem 15.1 bzw. 15.2 besitzen. Die Reaktoren R1 und R2 sind als Festbettreaktoren ausgeführt, und können sich in ihrer Bauform und der Art des verwendeten Katalysators unterscheiden. Bevorzugt kommen Rohrbündelreaktoren oder auch Plattenreaktoren zum Einsatz, um die bei der stark exothermen Methanisierung entstehende Wärme leichter abführen zu können. Zum anderen können die Stufen, u.a. aufgrund diverser Einstelleinrichtungen, die in dem Reaktorkonzept verwirklicht sind, mit unterschiedlichen Prozessparametern (Druck, Temperatur, Raumgeschwindigkeit) betrieben werden.

So ist zunächst durch einen Verdichter 7.1 der Gasdruck für das Eduktgas vor dem Eintritt in den Reaktor R1 einstellbar. Dem Verdichter 7.1 nachgeschaltet ist ein Wärmetauscher 6.1 angeordnet, mit dem das Eduktgas auf eine vorgebbare Temperatur, beispielsweise 270°C, gebracht werden kann. Mit 5.1 wird eine H₂O-Zugabeeinrichtung bezeichnet, die hinter dem Wärmetauscher 6.1 angeordnet ist, und über die der Wassergehalt des Eduktgases einstellbar ist. In den Abschnitt zwischen der H₂O-Zugabeeinrichtung 5.1 und dem Eintritt in den Reaktor R1 mündet eine Rezirkulationsleitung RZ, die in diesem Ausführungsbeispiel hinter dem zweiten Reaktor R2 abgeht und mittels der ein Teil des aus dem Reaktor 2 austretenden Gases vor den Reaktor 1 rezirkuliert werden kann.

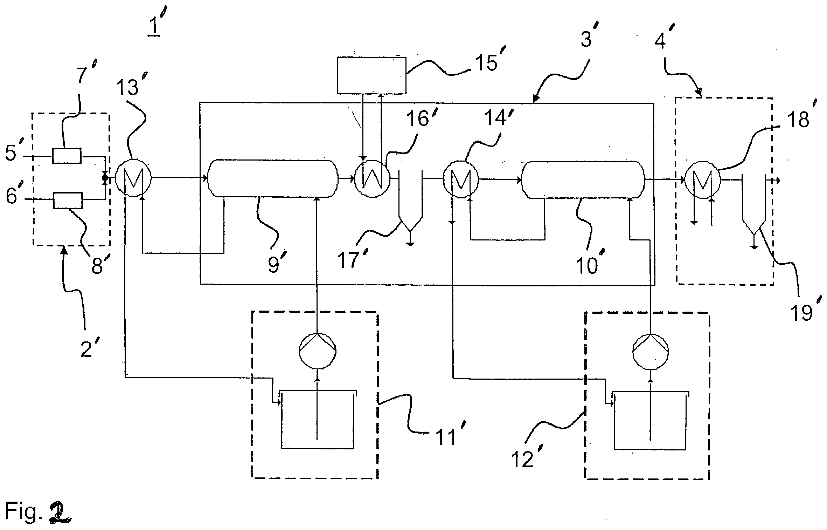

Dem Reaktor 1 nachgeschaltet ist ein weiterer Wärmetauscher 8.1, über den eine Abkühlung des aus dem Reaktor R1 austretenden Gases erreichbar ist. Es kann eine gewünschte Taupunkteinstellung vorgenommen werden, so dass an der mit 4.1 bezeichneten Stelle ein der Taupunkteinstellung entsprechender Anteil des im Gas enthaltenen Wassers abgeführt werden kann. Dazu kann ein aus dem Stand der Technik bekannter Kondensatableiter eingesetzt werden, siehe auch die genaueren Erläuterungen zu Fig. 2.

Weitere Einrichtungen bis zum Eintritt des Gases in den zweiten Reaktor R2 sind in der Reihenfolge des Gasflusses eine steuerbare Drosselung 9.1, ein Zwischenverdichter 7.2, ein Wärmetauscher 6.2 zum Wiedererwärmen des Gasgemisches sowie eine weitere H₂O-Zugabeeinrichtung 5.2, über die der Wassergehalt des in den Reaktor R2 eintretenden Gases noch weiter (wieder) erhöht werden kann.

Der zweite Reaktor R2 ist in der schematischen Darstellung von Fig. 1 schematisch gleich zum Reaktor R1 dargestellt, kann aber einen gegenüber dem Reaktor R1 einen Katalysator mit unterschiedlicher, insbesondere höherer Aktivität aufweisen und kann auch anderweitig anders ausgelegt sein.

Wie bereits oben erläutert, kann über die Rezirkulationsleitung RZ ein Teil des aus dem Reaktor 2 austretenden Gases vor den Reaktor 1 rezirkuliert werden. In der Rezirkulationsleitung RZ ist eine steuerbare Drosselung 90 vorgesehen, sowie ein Rezirkulationsverdichter 70. Letzterer ist optional und kann aufgrund der unten erläuterten Drucksteuerung über den Zwischenverdichter 7.2 auch entfallen. Die Rezirkulationsleitung RZ könnte auch zwischen den Reaktoren R1 und R2 abgezweigt werden, bevorzugt hinter dem Zwischenverdichter 7.2. Andernfalls sollte jedenfalls der Rezirkulationsverdichter 70 in RZ beibehalten bleiben.

Ähnlich wie beim Reaktor R1 ist auch dem Reaktor R2 eine Abkühleinrichtung in Form eines Wärmetauschers 8.2 zur Taupunkteinstellung mit entsprechender Wasserausscheidung 4.2 und eine steuerbare Drosselung 9.2 vorgesehen, bevor die Ausgangsstelle A erreicht wird. Allerdings ist vor der Drosselung 9.2 noch eine Umschalteinrichtung 3 eingebaut, mittels der das Gas auf einen dritten Reaktor R3 umgeleitet werden kann. Der Reaktor R3 vervollständigt in dem in Fig. 1 gezeigten Ausführungsbeispiel den stufenartigen Modulaufbau des Reaktorsystems und hat wie die Reaktoren R1 und R2 eine vorgelagerte Heizeinrichtung in Form eines Wärmetauschers 6.3 und eine H₂O-Zugabeeinrichtung 5.3 und nachgeschaltet eine Abkühleinrichtung in Form eines weiteren Wärmetauschers 8.3 zur erneuten Taupunkteinstellung mit entsprechender Wasserausscheidung 4.3 und einer steuerbaren Drosselung 9.3, bevor der im Falle des Einsatzes des dritten Reaktors R3 aktive Ausgang A3 erreicht wird. Auf den dritten Reaktor R3 kann auch vollständig verzichtet werden, sodaß das aus dem Reaktor R2 austretende Gas abgesehen von der anschließenden Entwässerung ohne weitere Aufbereitungsmaßnahme das Endproduktgas bilden kann.

Das Reaktorsystem wird von einer Steuereinrichtung 20 gesteuert, die drahtgebunden oder drahtlos ihre Steuerbefehle an die einzelnen Einstelleinrichtungen übermittelt und Sensordaten von in Fig. 1 nicht dargestellten Mess-Sensoren erhält, welche weiter unten genauer erläutert werden.

Zunächst werden jedoch einige bei der Methanisierung auftretenden Vorgänge erläutert, sowie Maßnahmen angegeben, um negativen Wirkungen dieser Vorgänge auf das Verfahren entgegenzuwirken.

Neben den in den eingangs erläuterten Gleichungen (1) bis (3) angegebenen Reaktionen finden auch andere Reaktionen statt, etwa die Umwandlung von Kohlenmonoxid in elementaren Kohlenstoff, der z. T. mit anderen Elementen rekombiniert auf der Katalysatoroberfläche im Reaktor abgeschieden werden und ggf. zu einer stetig fortschreitenden Deaktivierung des Katalysators führen kann. Um unerwünschte, die Aktivität des Katalysators beeinträchtigende Kohlenstoffabscheidungen im ersten Reaktor R1 zu vermeiden oder wenigstens zu verringern, kann H₂O in Form von Wasserdampf dem Eduktgas vor dessen Eintreten in den Reaktor 1 zugeführt werden, sofern nicht bereits ein ausreichender Wassergehalt über die Rezirkulation eingeführt wird. Dazu ist bei Bedarf die H₂O-Zugabeeinrichtung 5.1 vorgesehen.

Die Methanisierung ist eine stark exotherme Reaktion, so dass während der Methanbildung im Gas auch die Temperatur des Gases stark ansteigt (Ursache für die Hot Spots). So gelangt man zu Methangehalten durch die Methanisierung im ersten Reaktor R1 von ca. 65%-85vol%. Zum weiteren Erhöhen des Methangehalts wird in den Reaktoren R1, R2 und ggf. R3 ein gestaffeltes Temperaturniveau realisiert, welches das Gleichgewicht der Reaktionen zu hohen Methangehalten hin verschiebt. Um diese Temperaturniveaus zu erhalten, ist zum einen eine Reaktorkühlung für jede Reaktorstufe vorgesehen. Ein dazu vorgesehener Kühlkreislauf 15.1 (15.2, 15.3), der über eine Wärmetauschereinheit 18.1 (18.2, 18.3) reaktorseitig gekühlt wird und entsprechend die Reaktorwärme zur anderweitigen Verwendung aufnimmt, derart vorgesehen wird, dass der Kühlkreisstrom zu dem Reaktorgasstrom gleichläufig ist. Alternativ kann über einen gegenläufigen Kühlkreisstrom ein in Gasströmungsrichtung negativer Temperaturgradient erzeugt werden, was im Hinblick auf die anschließend ohnehin erfolgende Abkühlung energetisch vorteilhaft ist.

Für eine nochmals verbesserte Methanisierung im zweiten Reaktor R2 ist vorgesehen, über die Wasserentnahmestation mittels Taupunkteinstellung und Wasserabscheidung durch Kühlung des aus dem Reaktor R1 austretenden Gases einen dem eingestellten Taupunkt entsprechenden Teil des Wassers auskondensieren zu lassen, während ein weiterer Teil zur Verhinderung von Kohlenstoffablagerungen im zweiten Reaktor R2 im Gasstrom verbleibt, siehe hierzu auch die prinzipiellen Erläuterungen zu Fig. 2.

Insbesondere wenn das Eduktgas überwiegend oder im wesentlichen aus einem stöchiometrisch geeigneten CO₂/H₂-Gemisch besteht, entsteht aufgrund der bereits bei Eintritt in Reaktor 1 sehr rasch ablaufenden Reaktion bei den gewünschten Raumgeschwindigkeiten bereits soviel Reaktionswärme, dass eine ausreichende Reaktorkühlung über den Kühlkreislauf nicht mehr ausreicht. Dann können sich im Eingangsbereich des Reaktors die als "hot spots" bezeichneten lokal begrenzten Überhitzungsszonen ausbilden. Über Temperatursensoren kann die Ausbildung solcher "hot spots" erkannt werden und eine entsprechende Information an die Steuereinrichtung 20 übermittelt werden. Da der Katalysator den Temperaturbelastungen in den Überhitzungszonen auf längere Zeit nicht gewachsen ist, werden Gegenmaßnahmen zur Herabsetzung der Reaktionsgeschwindigkeit durchgeführt.

Eine dazu vorgesehene Maßnahme ist die Rezirkulation RZ eines Teils des z. B. aus dem Reaktor 2 austretenden, im wesentlichen schon ausreagierten Produktgases, das dem Eduktgas vor Eintritt in den ersten Reaktor R1 wieder zugemischt wird. Zur Beibehaltung des Druckniveaus kann der rezirkulierte Anteil zuvor über den Verdichter 70 verdichtet werden, der den Druckverlust über die Reaktoren R1 und R2 kompensiert. Während die Reaktionsgeschwindigkeit im Reaktor R1 aufgrund dieser Maßnahme sinkt, ist zudem zu berücksichtigen, die H₂O-Zugabe über die H₂O-Zugabeeinrichtung 5.1 aufgrund des rezirkulierten Methans anzupassen, oder ggf. zu stoppen.

Eine weitere Maßnahme, der Bildung von "hot spots" entgegenzuwirken oder aufgebaute "hot spots" zu beseitigen, besteht in der weiteren Einbremsung der Reaktionsgeschwindigkeit über eine Druckabsenkung des Eduktgases vor dem ersten Reaktor R1 über den Verdichter 7.1. Der Druck kann dabei stark abgesenkt werden, es ist lediglich noch ein geringfügiger Überdruck gegenüber der Umgebung erforderlich, um im Falle eines Lecks das Eintreten von Luft zu verhindern, z. B. kann ein absoluter Druck von 1,5 bar_{abs} gewählt werden.

In Folge verringert sich die Reaktionsgeschwindigkeit und die lokalen Überhitzungszonen bilden sich zurück. Mittels des Zwischenverdichters 7.2 kann der niedrige Druck im System wieder für den Reaktor R2 hochgefahren werden, in welchem das Problem der "hot spots" im wesentlichen nicht auftritt. Entsprechend kann die Nach-Methanisierung wieder bei höherem Druck, z.B. 7 bar, gefahren werden, welcher aufgrund der volumenreduzierenden Reaktion die Reaktionsgeschwindigkeit erhöht und das Gleichgewicht zu höheren Methangehalten verschiebt. In diesem Fall kann sogar ein Druckgefälle des Drucks hinter dem Reaktor R2 gegenüber dem Druck vor dem Reaktor R1 entstehen, so dass das rezirkulierte Gas nicht weiter verdichtet werden muss. Entsprechend kann auf den Einsatz des Rezirkulationsverdichters 70 verzichtet werden, und die Menge des ggf. feuchten rezirkulierten Gases wird allein über die steuerbare Drosselung 90 eingestellt.

Wie oben bereits erläutert, ist im Normalbetrieb (Base Case), beispielsweise bei voller Auslastung von einer aufgrund entsprechender Grundeinstellungen ausreichenden Produktgasqualität (Erfüllung des Kriteriums) nach Durchlaufen der beiden Reaktoren R1 und R2 auszugehen. Für den Fall, dass z.B. aufgrund einer schlechteren Eduktgasqualität oder einer nachlassenden Aktivität der Katalysatoren oder einem durchgeführten Lastwechsel das Kriterium nur mit den beiden Reaktoren R1 und R2 nicht erfüllbar ist, kann die Methanisierungsreaktion durch Zuschaltung des Reaktors R3 noch weitergeführt werden. Dies geschieht durch Umleitung des Gasstroms über die Umleiteinrichtung 3. Da der Reaktor R3 nur für die Endmethanisierung eingesetzt wird, kann dafür ein höchst aktiver Katalysator verwendet werden. Desweiteren ist über die vorgeschaltete Wasserentnahmesteuerung 8.2 und 4.2 eine erneute Abkühlung mit Taupunkteinstellung und entsprechender Wasserentnahme vorgesehen, so dass lediglich der für die Reaktorauslegung des Reaktors R3 erforderliche Wassergehalt im darin einströmenden Gas verbleibt, um wiederum unerwünschten Kohlenstoffabscheidungen am Katalysator entgegenzuwirken.

Unter Berücksichtigung der oben erläuterten Maßnahmen werden nun einzelne Steuerungen des Reaktorbetriebs erläutert, welche zum einen den mit der Erfindung erreichten intermittierenden Betrieb (Anpassung an wechselnde Last) optimieren. Weitere Optimierungen betreffen in der Einsparung der für das Verfahren aufgewendeten Energie sowie eine längere Ausnutzung des Katalysators.

Unter einem ersten Aspekt soll nur ein geringstmöglicher Verdichtungsaufwand beschrieben werden. Wie bereits oben erläutert, erlaubt die modulare Aufspaltung des Reaktorsystems in unabhängig voneinander einstellbare Reaktoren R1 und R2 u.a. über den Zwischenverdichter 7.2 eine willkürlich wählbare Druckeinstellung zwischen den einzelnen Reaktoren R1 und R2. Hinsichtlich der Druckeinstellung im Reaktor 1 über den Verdichter 7.1 wird der Reaktor R1 bei einem sehr geringen Druck (beispielsweise im Bereich von 1,5 bis 2 bar) betrieben, der jedoch um eine vorgegebene Sicherheitsmarge größer als der Umgebungsdruck eingestellt wird.

Durch die stark volumenverringernde Reaktion im ersten Reaktor wird bereits eine erhebliche Energieeinsparung erreicht, da der Zwischenverdichter 7.2 lediglich ein geringeres Gasvolumen auf einen im Reaktor 2 erwünschten höheren Druck zu bringen hat als es im Falle einer bereits vor dem Reaktor 1 vorgenommenen Verdichtung der Fall wäre. Darüber hinaus soll der Druck im Reaktor R2 unter Energieeinsparung ebenfalls noch soweit wie möglich abgesenkt werden, so dass die gewünschte Qualität des Produktgases (Erfüllung des ersten Kriteriums) gerade noch erreicht wird.

Bei festgelegtem Druck im Reaktor R1 erfolgt diese energieoptimierte Druckabsenkung für den Reaktor R2 automatisch über einen in die Steuerung 20 integrierten Regelkreis. Als das zu erfüllende Kriterium wird ein Mindestmethangehalt als Sollwert für den Regelkreis herangezogen. Der Istwert des Regelkreises ist dann der an der Ausgangsstelle gemessene Methangehalt des Produktgases. Bei positiver Regelabweichung zwischen gemessenem und erforderlichen Methangehalt wird als Steuergröße des Regelkreises der Druck vor dem Reaktor R2 weiter solange abgesenkt, bis der

Druck auf einen Wert geregelt ist, bei dem Istwert und Sollwert übereinstimmen und somit die gewünschte Produktqualität mit minimalem Energieaufwand erreicht wird. Dabei kann das gewünschte Kriterium noch eine Sicherheitsmarge gegenüber den Mindestanforderungen enthalten.

Aufgrund des Regelkreises erfolgt eine passende Druckeinstellung vor dem Reaktor 2 zudem auch dann, wenn eine Störung aufgrund einer schleichenden Degradation eines der Katalysatoren, einer geringfügigen Laständerung, oder Schwankungen z.B. in der Zusammensetzung des Kohlendioxidanteils des Eduktgases auftreten.

Eine weitere der optimierenden Regelung unterworfene Größe ist die Menge des rezirkulierten Gases basierend auf der Erkenntnis, dass der Energieaufwand am Zwischenverdichter 7.2 sich entsprechend der Menge des rezirkulierten Gases erhöht.

Diese weitere Regelung (Regelkreis 2) ist aufgrund der obigen Erläuterung der Problematik der "hot spots" an die Bedingung gekoppelt, dass die Temperatur im Reaktor R1 eine vorgegebene Grenztemperatur nicht überschreitet, welche ein Erfahrungswert mit Sicherheitsmarge unterhalb einer Katalysator-Grenztemperatur ist und dem Regelkreis 2 als Sollgröße aufgegeben wird. Als Istwert dient die im Reaktor 1 gemessene Temperatur, und die als Steuergröße fungierende rezirkulierte Gasmenge wird über die im Rezirkulationskreis RZ angeordnete Drosselung 90 eingestellt. Die rezirkulierte Gasmenge wird dabei reduziert, solange die gemessene Temperatur kleiner ist als die vorgegebene Solltemperatur. Wiederum werden auf diese Weise Störungen, welche zu einer Erhöhung der im Reaktor 1 herrschenden Temperatur führen und ggf. das Risiko der Bildung von "hot spots" erhöhen, wie die Erhöhung der umzusetzenden Eduktgasmenge pro Zeit durch eine automatisch geregelte (erhöhte) Rezirkulation ausgeglichen.

Über die Optimierung in Form einer geringstmöglichen Menge des rezirkulierten Gases wird zugleich im Reaktor R1 eine höchstmögliche Reaktortemperatur erreicht, so dass der energetische Wert der nutzbaren, über den zugehörigen Kühlkreislauf 15.1 und den Wärmetauscher 18.1 abgreifbaren Abwärme ebenfalls maximiert wird.

Wie oben bereits erläutert, sollte das Gas bei jedem Reaktoreintritt bevorzugt einen Mindestanteil an Wasser enthalten, um die Katalysatoren vor unerwünschten Kohlenstoffablagerungen zu schützen. Abgesehen von dieser Schutzwirkung wirkt sich die Zuführung von Wasser über die H₂O-Zugabeeinrichtungen 5.1 und 5.2 negativ auf die Energiebilanz des Verfahrens aus, da das Wasser aufbereitet, erwärmt und verdunstet werden muss und zum anderen einen über die spätere Verdichtung auszugleichenden Druckverlust erzeugt. Zum anderen wird das zugeführte Wasser nach den jeweiligen Reaktoren wieder auskondensiert, was ebenfalls die Verfahrensenergie erhöht.

Eine weitere Steuerung des Reaktorsystems sieht daher vor, den Wasseranteil bei jedem Reaktoreintritt so gering wie möglich zu halten. Bezüglich des Reaktors R1 soll das über die H₂O-Wasserzugabeeinrichtung 5.1 dampfförmig zugeführte Wasser minimiert werden. Als Grenzbedingung ist dafür vorgesehen, dass ein von mehreren Größen abhängiger und nach einer vorgegebenen Rechenvorschrift ermittelte Wassergehalt im Gas bei Eintritt in den Reaktor R1 nicht unterschritten wird. Zur Überprüfung der Erfüllung dieser Randbedingung wird die Gaszusammensetzung an jedem Reaktoreintritt kontinuierlich ermittelt und der Steuereinrichtung 20 übermittelt. Die Größen, über die der Soll-Wassergehalt errechnet wird, beinhalten eine oder mehrere von Eduktgaszusammensetzung, insbesondere den Kohlenwasserstoffgehalt unter Einrechnung der Rezirkulation, der Konvertierungsrate insbesondere von Reaktor R1, sowie Druck und Temperatur des Reaktors R1.

Diese Steuerung kann auch automatisch über einen weiteren Regelkreis (Regelkreis 3) durchgeführt werden, wobei als Istwert der indirekt bestimmte/gemessene Wassergehalt des Gases am Reaktoreintritt herangezogen wird und die Menge des über die H₂O-Zugabeeinrichtung 5.1 zudosierten Wassers die Stellgröße dieses dritten Regelkreises darstellt. Eine indirekte Bestimmung erhält man aus der Kenntnis des Eduktgases, des Produktgases sowie der rezirkulierten Gasmenge.

Auf ähnliche Weise soll auch der Wassergehalt des in Reaktor R2 eintretenden Gases möglichst gering gehalten werden. Gegenüber dem ersten Reaktor R1 ergibt sich jedoch der Unterschied, dass der Wassergehalt über zwei Maßnahmen veränderbar ist, zum einen über eine Wasserentnahme 4.1 mittels Taupunkteinstellung durch Abkühlung 8.1, und zum anderen über die Wasserzudosierung über die H₂O-Wasserzugabeeinrichtung 5.2. Somit stehen für eine entsprechende Regelung zwei Stellgrößen zur Verfügung. Idealerweise soll auf die Zuführung von Wasser über die H₂O-Wasser-zugabeeinrichtung 5.2 vollständig verzichtet werden, und die Regelung allein über die Taupunkteinstellung und Wasserentnahme nach dem Austritt des Gases aus Reaktor R1 erfolgen. Letztere ist allerdings der Randbedingung eines maximalen für den Zwischenverdichter 7.2 zulässigen Wassergehalt unterworfen. Sofern dieser Wassergehalt überschritten wird, endet die Regelung über die Taupunkteinstellung und Wasserabscheidung und die verbleibende Nachregelung erfolgt über die Wasserzugabe (5. 2). Als Sollwert für den Wassergehalt bei Eintritt in Reaktor R2 wird wiederum ein über eine vorgegebene Rechenvorschrift aus Druck im Reaktor R2, der Zwischenproduktgasbeschaffenheit (insbesondere deren Kohlenwasserstoffgehalt), und der Reaktortemperatur im Reaktor R2 ermittelte Wert herangezogen.

Aufgrund dieses Regelkreises 4 und des Regelkreises 3 wird automatisch ein Schutz der Katalysatoren erreicht und es werden Störgrößen unabhängig von deren Ursprung, die anderweitig eine weitere Degradierung der Katalysatoren bewirken würden, entgegengeregelt.

Eine weitere Optimierung ist für den Fall vorgesehen, dass zwar das zu erfüllende Kriterium z.B. den Methangehalt des Produktgases betrifft, das allerdings noch verschärft wird. Neben einer Anpassung auf dieses geänderte Kriterium zu dessen Erfüllung über den Druck im Reaktor R2 mittels des Zwischenverdichters 7.2 kann als weiterer Parameter die Zusammensetzung des Eduktgases verändert werden. Dies kann zum einen über ein geändertes Mischverhältnis des beispielsweise mittels Elektrolyse gewonnenen Wasserstoffs zu dem kohlendioxidhaltigen Anteil des Eduktgases geschehen, oder durch eine Variation des letzteren Anteils. Dazu kann beispielsweise über einen Anschluss an eine externe Gasleitung ein Anteil Biogas zugeführt werden, welches bereits selbst einen hohen Methananteil aufweist.

Eine erfindungsgemäße Optimierung des Reaktorbetriebs liegt in dessen Steuerung für einen intermittierenden Betrieb. Wie oben bereits erläutert, wird der Reaktor nicht nur im kontinuierlichen Betrieb eingesetzt, sondern erlaubt über durchführbare Lastwechsel innerhalb kurzer Zeit (d.h. dem Umsetzen unterschiedlicher Eduktgasmengen pro Zeiteinheit, die schnell variieren) auch eine Verarbeitung von z.B. aus regenerativen Quellen stammenden Überschussströmen.

Zunächst sei das Reaktorsystem auf eine maximale Gasumsetzung ausgelegt, für die energetisch sinnvolle Parameter gemäß den obigen Regelungen eingestellt sind. Von einer solchen Leistung bzw. der damit verbundenen Last kann der Reaktor innerhalb einer Umschaltzeit von weniger als 5 Minuten, insbesondere weniger als 2 Minuten mit einer niedrigeren Last betrieben werden, etwa 40% der Maximallast oder weniger, insbesondere 20% der Maximallast oder weniger. Auch ein vollständiges Abschalten ist denkbar. Diese Lastwechsel im Allgemeinen und schnelle Lastwechsel im Speziellen, welche für übliche Festbettreaktoren äußerst problematisch sind, werden durch den erfindungsgemäßen modularen Reaktoraufbau sowie dessen Steuerung entschärft.

Die Grundeinstellungen für einen ausgeregelten Normalbetrieb können beispielsweise wie folgt sein: Das Eduktgas wird zunächst auf eine Temperatur von 270°C vorgewärmt (6.1). In der ersten Methanisierungsstufe wird bei Temperaturen im Bereich von 270°C bis 550°C und einem Druck von 2 bar gearbeitet. Die Abkühlung nach Durchlauf der ersten Methanisierungsstufe folgt bis auf eine Temperatur zwischen 100°C und 135°C (8.1), wonach der kondensierte Wasseranteil abgeschieden wird (4.1). Die anschließende Kompression (7.2) erfolgt auf einen Druck von 7 bar. Soweit ein Wassergehalt des Gasgemisches noch unterhalb von 20% liegt, wird er auf 25% bis 35% erhöht (5.2), wobei eine Vorwärmung des rekomprimierten Gases auf ca. 280°C vorgenommen wird (6.2). Das Methanisieren in der zweiten Stufe erfolgt dann bei Temperaturen zwischen 350°C und 280°C und einem Druck von 7 bar.

Nun wird anhand eines ersten Beispiels beschrieben, wie die Steuerung des Verfahrens in dem Fall erfolgt, dass der Reaktor aus dem ausgeregelten Normalbetrieb mit nahezu maximaler Leistung auf 20% dieser Leistung heruntergefahren werden soll, beispielsweise da die aus regenerativen Energien gewonnene elektrische Leistung für den Elektrolyseur zur Wasserstoffherstellung aufgrund von Windstille und/oder geringer Sonneneinstrahlung deutlich abgesunken ist. Andernfalls ist denkbar, dass die Erzeugung von Wasserstoff heruntergefahren wird, da der dazu erforderliche Strom am Strommarkt nur zu unrentablen Preisen erhältlich ist.

Dann erfolgt aufgrund der Reduzierung des Eduktgasstroms eine verringerte Raumgeschwindigkeit in den Reaktoren, deren Katalysatoren werden entlastet und erlauben noch höhere Methanisierungsgrade. Entsprechend kann über die oben beschriebenen Regelungen, insbesondere die Regelkreise 1 und 2 das Kriterium eines Mindestmethananteils auch mit niedrigerem Energieaufwand erfüllt werden, so dass bei sinkender Leistung aufgrund der Regelkreise entsprechende Energieeinsparungen automatisch erfolgen.

Im umgekehrten Fall, wenn der Steuereinrichtung 20 signalisiert wird, dass das Reaktorsystem von einer vergleichsweise niedrigen Last von z.B. 20% der Maximallast auf volle Leistung hochgefahren werden soll, werden die auf die Optimierung des Energieverbrauchs gerichteten Steuerungen und Regelungen zunächst ausgesetzt, da es an Energie im Prozess mangelt und die schnelle Bereitstellung der erforderlichen Reaktionswärme zur Laststeigerung wichtiger ist als mögliche Energieeinsparungen während der Laststeigerung. Es kann zu Regelungszwecken vorbereitend und vorübergehend ein schärferes Kriterium vorgegeben werden.

Die Steuereinrichtung 20 steuert in Vorbereitung des Lastwechsels eine oder mehrere der Einstelleinrichtungen, insbesondere die Einrichtungen 7.1, 6.1, 7.2, 90 derart, dass Parameter, welche die Freisetzung von Reaktionswärme beeinflussen, auf diese Freisetzung begünstigende Werte hin verändert werden. Es wird der Druck für Reaktor R1 und/oder der Druck für Reaktor R2 erhöht und ggf. das Eduktgas zusätzlich auf höhere Werte aufgeheizt. Im Lastwechsel kann dann zwar eine Nichteinhaltung des verschärften Kriteriums (z.B. CH₄-Gehalt) erfolgen, aber das ursprüngliche Kriterium bleibt eingehalten.

Eine Gegensteuerung auf die Reaktionsgeschwindigkeit und Freisetzung der Reaktionswärme hemmende Parametereinstellung beginnt, sobald sich insbesondere im Reaktor R1 lokal zu hohe Temperaturen aufbauen und sich "hot spots" ausbilden. Nach Einpendeln auf die neue Leistung (im Erreichen stationärer Reaktionsbedingungen unter der neuen Last) kann die auf eine Optimierung des Energieverbrauchs gerichtete Steuerung/Regelung wieder aufgenommen, und ggf. das verschärfte Kriterium wieder zurückgesetzt werden.

Eine zusätzliche Möglichkeit, eine Laststeigerung in möglichst kurzer Zeit zu bewältigen, liegt in der Zuschaltung des Reaktors R3. Im unter Energieoptimierung gesteuerten Normalbetrieb des Reaktorsystems wird dieser Reaktor R3 bevorzugt nur dann zugeschaltet, wenn die oben erläuterten weiteren Möglichkeiten zur Aufrechterhaltung der erforderlichen Produktgasqualität (Erfüllung des Kriteriums) bereits ausgeschöpft sind. In der in Fig. 1 gezeigten Darstellung ist der Reaktor R3 zuschaltbar (3) vorgesehen und kann durch seinen modulartigen Anschluss den Reaktoren R1 und R2 nachgeschaltet werden. In einer alternativen, nicht dargestellten Ausführungsform der Erfindung wird allerdings auch daran gedacht, auf den Reaktor R3 vollständig zu verzichten, und eine höhere Flexibilität ggf. durch eine großzügigere Dimensionierung des zweiten Reaktors R2 anzustreben.

Der modulartige Aufbau des Reaktorsystems aus einzelnen Reaktoren, die im Baureihensystem aneinander angekoppelt werden können und als Teile eines Baureihensystems auch einfacher industriell in kostengünstiger Serienfertigung hergestellt werden können, erlaubt somit eine höhere Flexibilität hinsichtlich der Verarbeitung unterschiedlicher Eduktgase und des Erreichens unterschiedlicher Leistungsbereiche. Des Weiteren wird über die individuelle Ansteuerbarkeit der einzelnen Reaktoren, insbesondere über die Einflussnahme auf das zwischen den beiden Reaktoren R1 und R2 strömende Gas die erläuterte flexiblere Steuerung des Systems ermöglicht, und insbesondere eine energieoptimierte Steuerung.

Im Folgenden werden noch mit Bezug auf Fig. 2 Einzelheiten hinsichtlich der Taupunkteinstellung oder der Gasmischung beschrieben, die sich grundsätzlich auch auf die anhand Fig. 1 beschriebenen Ausführungsform übertragen lassen, während die im Folgenden genannten Parameterwerte nicht als Einschränkung der Erfindung zu verstehen sind, sondern als mögliche Wahl.

Das Reaktorsystem 1' besteht aus einer Mischeinheit 2' zum Mischen von Kohlendioxid (CO₂) und Wasserstoff (H₂) in einem vorbestimmten molaren Verhältnis SN, einer Syntheseeinheit 3' zum Methanisieren des Kohlendioxids und Wasserstoff enthaltenden Gasgemisches sowie einer Trockeneinheit 4', in der dem in der Syntheseeinheit 3' methanisierten Gasgemisch Wasser entzogen wird.

Die Mischeinheit 2' weist eine Zuführleitung 5' für Kohlendioxid (CO₂) und eine Zuführleitung 6' für Wasserstoff (H) auf. In den Zuführleitungen 5', 6' ist jeweils ein Massendurchflussregler (engl. Mass Flow Controller = MFC) 7', 8' vorgesehen, mit dem der durch die jeweilige Zuführleitung 5', 6' fließende Gasstrom gesteuert werden kann. Stromabwärts von den Massendurchflussreglern 7', 8' werden die beiden Zuführleitungen 5', 6' in eine Gasleitung zusammengeführt.

Die Syntheseeinheit besteht wie in Fig. 2 dargestellt aus zwei hintereinander angeordneten Reaktorstufen 9' und 10', die jeweils einen an sich bekannten nickelhaltigen Katalysator, aufweisen.

Die beiden Reaktorstufen 9' und 10' sind jeweils mit einem geeigneten Kühlmedium von außen gekühlt. Das dabei verwendete Kühlmedium wird jeweils in einem Kreislauf an der Außenwand der jeweiligen Reaktorstufe 9', 10' vorbeigeleitet, sodass das Kühlmedium einen Teil der durch die Exothermie der Methanisierungsreaktion (3) entstehenden Wärme von der jeweiligen Reaktorstufe 9', 10' aufnehmen kann. In dem Kühlmediumkreislauf der ersten und der zweiten Reaktorstufe 9' und 10' ist jeweils ein Kühlmediumtemperiergerät 11', 12' enthalten, das das Kühlmedium auf eine vorbestimmte Temperatur bringt, bevor es an der Außenwand der Reaktorstufe 9', 10' vorbeigeleitet wird.

Das von dem Kühlmediumtemperiergerät 11', 12' kommende Kühlmittel wird an der ersten bzw. an der zweiten Reaktorstufe 9', 10' entgegen der Flußrichtung des Gasgemisches, d. h. vom Ausgang der Reaktorstufe 9', 10' zum Eingang der Reaktorstufe 9', 10', vorbeigeführt. Dadurch wird erreicht, dass der Kühleffekt am Ausgang der Reaktorstufe 9', 10' maximal ist und zum Eingang der Reaktorstufe 9', 10' hin stetig abnimmt, da die Temperatur des Kühlmittels aufgrund der von dem Gasgemisch aufgenommenen Wärmeenergie zum Eingang hin zunimmt und damit der Temperaturunterschied zwischen der Temperatur des Gasgemisches und der Temperatur des Kühlmediums abnimmt.

Das von der Reaktorstufe 9', 10' kommende, durch die Exothermie der Methanisierungsreaktion (3) aufgewärmte Kühlmittel wird, bevor es zurück zu dem Temperiergerät 11', 12' geführt wird, zuvor dazu verwendet, in einem Wärmetauscher 13', 14' das Gasgemisch vor dem Eintritt in die erste Reaktorstufe 9' bzw. vor dem Eintritt in die zweite Reaktorstufe 10' vorzuwärmen. Von dem Wärmetauscher 13', 14' kommendes Kühlmittel wird beim Kühlmittelkreislauf der ersten als auch beim Kühlmittelkreislauf der zweiten Reaktorstufe jeweils zu dem Temperiergerät 11', 12' zurückgeführt.

Zwischen den beiden Reaktorstufen 9', 10' weist die Syntheseeinheit 3' eine Vorrichtung zur Taupunkteinstellung des Gasgemisches nach dem Austritt aus der ersten Reaktorstufe 9' und vor dem Wärmetaucher 14' zum Vorwärmen des in die zweite Reaktorstufe 10' eintretenden Gasgemisches auf. Diese Vorrichtung zur Taupunkteinstellung besteht aus einem Kühlmittelkreislauf mit einem Kühler 15' und einem Wärmetauscher 16' zum Abkühlen des aus der ersten Reaktorstufe 9' austretenden Gasgemisches, sowie einem Kondensatableiter 17', der das durch die Abkühlung des Gasgemisches kondensierte Wasser aus dem Synthesesystem 3' bzw. dem Reaktorsystem 1' entfernt.

Nach dem Austritt aus der zweiten Reaktorstufe 10' wird das methanisierte Gasgemisch in eine Trockeneinheit 4' eingeleitet. Diese Trockeneinheit 4' besteht ähnlich wie die Vorrichtung zur Taupunkteinstellung aus einem Kühlmittelkreislauf mit einem Kühler (nicht dargestellt) und einem Wärmetauscher 18' zum Abkühlen des aus der zweiten Reaktorstufe 10' austretenden Gasgemisches, sowie einem Kondensatableiter 19', der das durch die Abkühlung des Gasgemisches kondensierte Wasser aus der Trockeneinheit 4' bzw. aus dem Reaktorsystem 1' entfernt.

In der Mischeinheit 2' wir durch entsprechendes Steuern der Massendurchflussregler 7', 8' reines Kohlendioxid (CO₂), das z. B. aus Luft oder Biogas gewonnen wird, und reiner Wasserstoff (H₂), der z. B. durch Elektrolyse aus Wasser gewonnen wird, stöchiometrisch gemischt. Das in der Mischeinheit 2' erzeugte Gasgemisch weist damit bei der vorliegenden Ausführungsform im Wesentlichen nur Kohlendioxid und Wasserstoff auf.

In diesem Ausführungsbeispiel wird das Gasgemisch durch Vorwärmung in dem ersten Wärmetauscher 13' vor der ersten Reaktorstufe 9' auf eine Temperatur von etwa 270°C vorgewärmt, bevor es in die erste Reaktorstufe 9' eingeleitet wird. Nach dem Einleiten des so vorgewärmten Gasgemisches führt die Exothermie der Methanisierungsreaktion, die durch den Katalysator in Gang gesetzt wird, zu einer schnellen Erwärmung des Gasgemisches auf eine maximale Temperatur von etwa 450°C. Danach fällt die Temperatur des Gasgemisches zum Ausgang der ersten Reaktorstufe 9' hin auf einen Wert von etwa 300°C ab. Nach dem Austritt aus der ersten Reaktorstufe 9' wird das Gasgemisch in der Vorrichtung zur Taupunkteinstellung auf eine Temperatur von etwa 120°C abgekühlt und das dabei kondensierte Wasser wird mit dem Kondensatableiter 17' aus der Syntheseeinheit 3' bzw. dem Reaktorsystem 1' entfern. Nach der Taupunkteinstellung wird das Gasgemisch mit dem Wärmetauscher 14' auf eine Temperatur von etwa 260°C vorgewärmt. Die Temperatur des Gasgemisches steigt nach dem Einleiten des Gasgemisches in die zweite Reaktorstufe 10' nur leicht auf etwa 280°C an und fällt zum Ausgang hin um etwa 10°C auf eine Temperatur von 270°C leicht ab.

Im Anschluss an die Methanisierung in der ersten und der zweiten Reaktorstufe 9', 10' wir dem methanisierten Gasgemisch das durch die Methanisierungsreaktion entstehende H₂O in der Trocknungseinheit 4' entzogen, indem das Gasgemisch in dem Wärmetauscher 18' auf eine Temperatur um etwa 30°C abgekühlt wird und das dabei kondensierende Wasser mit dem Kondensatableiter 19' abgeführt wird.

Der Druck in dem Reaktorsystem 1' bzw. in der Syntheseeinheit 3' wird gemäß dem Ausführungsbeispiel auf 6 bar eingestellt. Die Massenflüsse von Kohlendioxidgas und Wasserstoffgas wurden bei dem vorliegenden Ausführungsbeispiel mit den Massendurchflussreglern 7', 8' derart eingestellt, dass die Raumgeschwindigkeit in der ersten Reaktorstufe einen Wert von 3500/h und in der zweiten Reaktorstufe einen etwas geringeren Wert von 2500/h aufweist.

Der molare Wassergehalt in dem Gasgemisch lag bei dem vorliegenden Ausführungsbeispiel nach der Taupunkteinstellung zwischen 30% und 35%.

Nach der Trocknung in der Trocknungseinheit 4 beträgt der molare Wassergehalt des Gasgemisches einen dem Taupunkt von 30°C entsprechenden Wert.

Der Umsatz des Kohlendioxids in der ersten Reaktorstufe 9' beträgt bei dem Ausführungsbeispiel etwa 95%, de Umsatz des Kohlendioxids in der zweiten Reaktorstufe 10' beträgt bei dem Ausführungsbeispiel etwas über 90%, sodass der Methangehalt in dem methanisierten Gasgemisch am Ende etwa 99% beträgt.

Das so hergestellte Gasgemisch mit hohem Methangehalt kann unmittelbar als Kraftstoff für Fahrzeuge verwendet werden, oder kann unmittelbar in das Erdgasnetz eingespeist werden.

Es sind jedoch auch abgewandelte Formen des Reaktorsystems 1' und des damit durchgeführten Verfahrens gemäß der Erfindung möglich.

So wurde das Reaktorsystem 1 mit zwei Reaktorstufen 9', 10' beschrieben. Es ist jedoch auch möglich, weitere Reaktorstufen vorzusehen, falls eine noch vollständigere Methanisierung notwendig ist.

Die Methanisierung eines Kohlendioxid und Wasserstoff enthaltenden Gases ist nicht nur bei 6 bar sondern auch bei anderen Drücken möglich. Bevorzugt wird jedoch ein Druck in dem Bereich 2 bis 15 bar. Besonders vorteilhaft ist ein Druck in dem Bereich 2 bis 8 bar.

Es wurde oben eine bestimmte Temperaturführung beschrieben. Andere Temperaturführungen sind denkbar. So könnten andere Temperaturen für die Vorwärmung des Gasgemisches vor Einleiten in die erste bzw. zweite Reaktorstufe 9', 10' gewählt werden. Je nach verwendetem Katalysator könnte das Verfahren auch ohne Vorwärmung des Gasgemisches vor dem Einleiten desselben in die erste bzw. die zweite Reaktorstufe 9', 10' durchgeführt werden. Dadurch würde der apparative Aufwand verringert.

Das Verfahren wurde im Ausführungsbeispiel mit Temperaturen in der ersten Reaktorstufe 9' von 300°C bis 450°C beschrieben. In diesem Bereich erfolgt die Methanisierung besonders effizient. Es ist jedoch auch möglich, die Methanisierung in der ersten Reaktorstufe bei anderen Temperaturen durchzuführen, wobei Temperaturen im Bereich von 300°C bis 600°C gewählt werden, bei denen eine effiziente Methanisierung erreicht wird. Die Nachmethanisierung in der zweiten Reaktorstufe wurde so beschrieben, dass sie bei Temperaturen im Bereich von 270°C bis 280°C durchgeführt wird. In diesem Bereich erfolgt die Nachmethanisierung besonders effizient. Es ist jedoch auch möglich, die Nachmethanisierung bei anderen Temperaturen durchzuführen, wobei Temperaturen im Bereich von 250°C bis 300°C Vorteile bieten können.

Zur Taupunkteinstellung wurde das Abkühlen des Gasgemisches auf eine Temperatur von 120°C beschrieben. Je nach verwendetem Druck kann jedoch eine andere Temperatur notwendig sein, um den Taupunkt optimal einzustellen. Bei Drücken zwischen 2 und 15 bar liegt die Temperatur, auf die das Gasgemisch zur Taupunkteinstellung abgekühlt werden muss, um einen molaren Wassergehalt von 30% bis 50% einzustellen in einem Bereich von 80°C bis 160°C. Bei Drücken im bevorzugten Bereich 2 bis 8 bar liegt diese Temperatur in einem Bereich von 86°C bis 128°C. Vorteilhafterweise wird die Taupunkteinstellung so durchgeführt, dass der molare Wassergehalt in einem Bereich von 30% bis 50%, besonders bevorzugt von 30% bis 35% liegt.

Bei dem obigen Ausführungsbeispiel wurden bestimmte Raumgeschwindigkeiten beschrieben, die für eine effiziente Methanisierung besonders vorteilhaft sind. Jedoch sind auch andere Raumgeschwindigkeiten denkbar, vorzugsweise liegt die Raumgeschwindigkeit des Gasgemisches in der ersten Reaktorstufe 9' in einem Bereich von 2000/h bis 8000/h. Besonders vorteilhaft ist eine Raumgeschwindigkeit in einem Bereich von 2000/h bis 6000/h. Die Raumgeschwindigkeit in der zweiten Reaktorstufe 10' liegt in einem Bereich von 1000/h bis 6000/h. Besonders vorteilhaft ist eine Raumgeschwindigkeit im Bereich von 1500/h bis 4000/h.

Bei dem oben angegebenen Ausführungsbeispiel wurde als Kohlendioxid und Wasserstoff enthaltendes Gasgemisch ein Gasgemisch verwendet, das im Wesentlichen nur aus Kohlendioxid und Wasserstoff besteht. Das für die Methanisierung verwendete Gasgemisch kann jedoch auch weitere Bestandteile enthalten. Gegebenenfalls müssen vor dem Einleiten in die erste Reaktorstufe 9' Katalysatorgifte wie z. B. Schwefelverbindungen entfernt werden. Das Kohlendioxid und Wasserstoff enthaltende Gasgemisch kann z. B. Biogas sein, das zusätzlich zu Kohlendioxid und Wasserstoff schon einen bestimmten Methananteil besitzt und einen Kohlenmonoxidanteil von weniger als 0,1% aufweist. Das molare Verhältnis zwischen Kohlendioxid und Wasserstoff kann dabei wiederum durch Zumischen von Wasserstoff erfolgen.

Weiter wurde bei dem oben beschriebenen Ausführungsbeispiel ein stöchiometrisches Mischen von Kohlendioxid und Wasserstoff beschrieben. Jedoch ist es auch möglich, ein davon abweichendes Mischungsverhältnis von Kohlendioxid und Wasserstoff zu verwenden.

Es wurde schließlich bei dem oben angegebenen Ausführungsbeispiel beschrieben, das methanisierte Gasgemisch durch Abkühlen auf 30°C und Abführen des kondensierten Wassers in der Trockeneinheit 4' zu trocknen. Die Trocknung des methanisierten Gasgemisches kann aber auch mit anderen bekannten Verfahren durchgeführt werden. Darüber hinaus kann die Trocknung je nach Anforderungen an das Ausgangsgasgemisch angepasst und gegebenenfalls auch ganz weggelassen werden.

Die Erfindung ist nicht auf die in der detaillierten Figurenbeschreibung erläuterte Ausführungsform eingeschränkt. Vielmehr können die in der obigen Beschreibung sowie in den Ansprüchen offenbarten Merkmale der Erfindung sowohl einzeln als auch in beliebiger Kombination für die Verwirklichung der Erfindung in ihren verschiedenen Ausführungsformen wesentlich sein.

## Patentansprüche

1. Verfahren zur Herstellung eines methanreichen Produktgases im intermittierenden Betrieb zwischen einem Normalzustand und einer niedrigeren Auslastung von 60% oder weniger der maximalen Auslastung, bei dem man ein Wasserstoff und Kohlendioxid enthaltendes Eduktgas in wenigstens zwei Stufen (R1, R2) unter Einfluss eines einstellbaren Drucks und einer einstellbaren Menge eines abgezweigten und vor der ersten Stufe rezirkulierten Gases katalytisch methanisiert und den Methangehalt des Produktgases überwacht, **dadurch gekennzeichnet, dass**
in dem Zustand niedriger Auslastung und bei der hierbei pro Zeiteinheit strömenden Menge des Eduktgasstroms der Methangehalt des Produktgases mehr als 90 vol.% beträgt und man bei Erhöhung der pro Zeiteinheit strömenden Eduktgasmenge im Übergang von dem Zustand niedriger Auslastung in den Normalzustand zur Wahrung dieses Methangehalts im Produktgas die Reaktionsrate durch Erhöhung des Drucks erhöht, sowie in einer Gegensteuerung zur Vermeidung von sich dadurch bildenden lokal begrenzten Überhitzungszonen die Menge des rezirkulierten Gases erhöht.

2. Verfahren nach Anspruch 1, bei dem wenigstens ein Teil des in dem Eduktgas enthaltenen Wasserstoffs insbesondere mittels Elektrolyse unter Abnahme einer elektrischen Last bereitgestellt wird, und die im Zustand niedriger Auslastung pro Zeiteinheit strömende Menge des Eduktgasstroms durch die abgenommene Last bedingt ist.

3. Verfahren nach einem der vorhergehenden Ansprüche, bei dem die Methanisierung unter Einfluss wenigstens eines weiteren Parameters aus einer Gruppe erfolgt, die aufweist: den Wassergehalt des Gases vor der ersten Methanisierungsstufe; den Wassergehalt des Gases vor der zweiten Methanisierungsstufe; eine nach der ersten Methanisierungsstufe aus dem Gasgemisch entfernte Wassermenge; eine dem Erwärmen des Gases vor der ersten und/oder zweiten Methanisierungsstufe dienende Heizleistung; eine Einbeziehung JA/NEIN einer dritten Methanisierungsstufe; die Zusammensetzung des Eduktgases.

4. Verfahren nach einem der vorhergehenden Ansprüche, bei der die Änderung des Drucks und/oder der Menge des rezirkulierten Gases automatisch und insbesondere über eine Regelung erfolgt.

5. Verfahren nach einem der vorhergehenden Ansprüche, bei dem die Änderung des Drucks und/oder der Menge des rezirkulierten Gases einer weiteren einzuhaltenden Randbedingung unterliegt.

6. Verfahren nach Anspruch 5, bei dem die weitere Randbedingung aus der Gruppe ausgewählt ist, die aufweist: Maximaltemperatur des Katalysators in der ersten und/oder zweiten Methanisierungsstufe; Mindest- und/oder Maximalwassergehalt des Gases vor der ersten und/oder zweiten Methanisierungsstufe; Mindest- und/oder Maximalmenge des nach dem ersten Methanisierungsschritt entfernten Wassers; Erfüllung eines die Zusammensetzung des hergestellten Gasgemisches betreffenden Kriteriums.

7. Verfahren nach einem der vorhergehenden Ansprüche, bei dem die Einhaltung der Maximaltemperatur und ggf. der weiteren Randbedingung über einen entsprechend zugeordneten Regelkreis gesteuert und insbesondere darüber überwacht wird.

8. Verfahren nach einem der vorhergehenden Ansprüche, bei dem die Gegensteuerung vorgenommen wird, sobald ein Überschreiten einer vorgegebenen Temperatur in der ersten Methanisierungsstufe festgestellt wird.

9. Verfahren nach Anspruch 8, bei dem eine energiesparende Steuerung vorgenommen wird, sobald bezüglich des geänderten Eduktgasstroms ein stationärer Zustand erreicht ist.

10. Reaktorsystem zur Herstellung eines methanreichen Produktgases aus einem Wasserstoff und Kohlendioxid aufweisenden Eduktgas, mit
wenigstens zwei hintereinandergeschalteten, einen Katalysator aufweisenden Reaktorstufen (R1, R2),
einer Überwachungseinrichtung zur Überwachung des Methangehalts des Produktgases,
einer Rezirkulationseinrichtung (RZ) zur Rückführung eines Teils des Gases, insbesondere des aus der zweiten Reaktorstufe ausgetretenen Gases an eine vor der ersten Reaktorstufe gelegene Stelle,
einer an die Überwachungseinrichtung gekoppelten Steuereinrichtung (20), und
wenigstens einer von der Steuereinrichtung (20) gesteuerten Einstelleinrichtung (7.1, 7.2, 70) zur Einstellung des Drucks bei der Methanisierung,
**dadurch gekennzeichnet, dass** das Reaktorsystem für einen intermittierenden Betrieb zwischen einem Normalzustand und einer niedrigeren Auslastung von 60% oder weniger der maximalen Auslastung ausgelegt und durch die Steuerung der Steuereinrichtung dazu betreibbar ist, ein Verfahren nach einem der Ansprüche 1 bis 9 durchzuführen.

11. Reaktorsystem nach Anspruch 10, bei dem eine zweite Einstelleinrichtung eine Einrichtung (8.1, 4.1) zum Entfernen wenigstens eines Teils des nach der ersten Reaktorstufe im Gas enthaltenen Wassers ist.

12. Reaktorsystem nach Anspruch 11, bei dem die zweite Einstelleinrichtung eine vorgebbare Taupunkteinstellung erlaubt.

13. Reaktorsystem nach einem der Ansprüche 10 bis 12, bei dem eine dritte Einstelleinrichtung (5) zum Zuführen von Wasser vor einer oder mehreren der Reaktorstufen vorgesehen ist.

14. Reaktorsystem nach einem der Ansprüche 10 bis 13, bei dem eine vierte Einstelleinrichtung (6) zum Erwärmen des in einer oder mehreren Reaktorstufen eingeleiteten Gases vorgesehen ist.

15. Reaktorsystem nach einem der Ansprüche 10 bis 14, bei dem die Rezirkulationseinrichtung einen Verdichter (70) und/oder eine steuerbare Drosselung (90) aufweist.

16. Reaktorsystem nach einem der Ansprüche 10 bis 15, das weiter eine über die Steuereinrichtung zuschaltbare dritte Reaktorstufe (R3) aufweist.

17. Reaktorsystem nach einem der Ansprüche 10 bis 16, das an eine Einrichtung zur Herstellung von Wasserstoff mittels elektrischer Energie ankoppelbar und insbesondere angekoppelt ist.

18. Reaktorsystem nach einem der Ansprüche 10 bis 17, das an eine Einrichtung zur Herstellung von Kohlendioxid, insbesondere über eine Gaswäsche, ankoppelbar und insbesondere angekoppelt ist.

19. Reaktorsystem nach einem der Ansprüche 10 bis 17, das einen Anschluss zum Zuführen eines als Teil des Eduktgases verwendbaren Gases, insbesondere eines Biogases aufweist.

20. Reaktorsystem nach einem der Ansprüche 10 bis 19, bei dem eine oder mehrere der Einstelleinrichtungen (7.2, 90, 5.1, 8.1, 5.2) über einen Regelkreis steuerbar sind.

## Claims

1. A method for manufacturing a methane-rich product gas in intermittent operation between a normal state and a lower capacity utilisation of 60% or less of the maximum capacity utilisation, wherein a starting gas containing hydrogen and carbon dioxide is catalytically methanated in at least two stages (R1, R2) under the influence of a settable pressure and a settable quantity of a gas diverted and recirculated upstream of the first stage, and the methane content of the product gas is monitored, **characterised in that**
in the state of low capacity utilisation and at the quantity of starting gas flowing per unit of time the methane content of the product gas is more than 90 vol.% and, when the quantity of starting gas flowing per time unit is increased in the transition from the low capacity utilisation state to the normal state to maintain this methane content in the product gas, the reaction rate is increased by increasing the pressure and the quantity of recirculated gas is increased as a countermeasure to avoid the locally limited overheating zones created as a result.

2. The method according to claim 1, wherein at least a portion of the hydrogen contained in the starting gas is provided in particular by means of electrolysis, by consuming an electric load, and the quantity of starting gas flowing per unit of time in the low capacity utilisation state is determined by the consumed load.

3. The method according to one of the preceding claims, wherein methanation is performed under the influence of at least one further parameter from a group comprising: the water content of the gas upstream of the first methanation stage; the water content of the gas upstream of the second methanation stage; a quantity of water removed from the gas mixture downstream from the first methanation stage; a heating output used to heat the gas upstream of the first and/or second methanation stage; an inclusion YES/NO of a third methanation stage; the composition of the starting gas.

4. The method according to one of the preceding claims, wherein the change in pressure and/or quantity of gas to be recirculated is performed automatically and in particular by means of an adjustment.

5. The method according to one of the preceding claims, wherein the change in pressure and/or quantity of recirculated gas is subject to a further general condition to be met.

6. The method according to claim 5, wherein the further general condition is selected from the group comprising: the maximum temperature of the catalyser in the first and/or second methanation stage; minimum and/or maximum water content of the gas upstream of the first and/or second methanation stage; minimum and/or maximum quantity of the water removed downstream of the first methanation step; fulfilment of a criterion relating to the composition of the gas mixture produced.

7. The method according to one of the preceding claims, wherein fulfilment of the maximum temperature and, where applicable the further general condition, is controlled by means of an appropriately assigned adjustment circuit and in particular is monitored by means of this adjustment circuit.

8. The method according to one of the preceding claims, wherein the countermeasure is implemented as soon as a predetermined temperature is found to have been exceeded in the first methanation stage.

9. The method according to claim 8, wherein an energy-saving measure is implemented as soon as a steady state is reached regarding the changed starting gas flow.

10. A reactor system to produce a methane-rich product gas from a starting gas containing hydrogen and carbon dioxide, comprising
at least two reactor stages (R1, R2) connected one behind the other and comprising a catalyser,
a monitoring device for monitoring the methane content of the product gas,
a recirculation device (RZ) for the recycling of a portion of the gas, in particular of the gas exiting the second reactor stage, to a site located upstream of the first reactor stage.
a control device (20) coupled to the monitoring device, and
and at least one setting device (7.1, 7.2, 70) controlled by the control device (20), for setting the pressure during methanation,
**characterised in that** the reactor system is designed for intermittent operation between a normal state and a lower capacity utilisation of 60% or less of the maximum capacity utilisation and, due to the control of the control device, can be operated so as to perform a method according to one of claims 1 to 9.

11. The reactor system according to claim 10, wherein a second setting device is a device (8.1, 4.1) for removing at least a portion of the water contained in the gas downstream of the first reactor stage.

12. The reactor system according to claim 11, wherein the second setting device allows for a preselectable dewpoint setting.

13. The reactor system according to one of claims 10 to 12, wherein a third setting device (5) is provided for feeding water upstream of one or more of the reactor stages.

14. The reactor system according to one of claims 10 to 13, wherein a fourth setting device (6) is provided for heating the gas introduced in one or more reactor stages.

15. The reactor system according to one of claims 10 to 14, wherein the recirculation device has a compressor (70) and/or a controllable flow restrictor (90).

16. The reactor system according to one of claims 10 to 15, which further has a third reactor stage (R3) which is connectable via the control device.

17. The reactor system according to one of claims 10 to 16, which can be coupled and in particular is coupled to a device for generating hydrogen by means of electrical energy.

18. The reactor system according to one of claims 10 to 17, which can be coupled and in particular is coupled to a device for producing carbon dioxide, particularly via a gas scrubber.

19. The reactor system according to one of claims 10 to 17, which has a connection for feeding a gas, particularly a biogas, usable as a portion of the starting gas.

20. The reactor system according to one of claims 10 to 19, wherein one or more of the setting devices (7.2, 90, 5.1, 8.1, 5.2) are controllable via an adjustment circuit.

## Revendications

1. Procédé permettant de produire un gaz de production riche en méthane en fonctionnement intermittent entre un état normal et un taux d'utilisation réduit de l'ordre de 60 %, voire moins, du taux d'utilisation maximal, dans lequel on effectue une méthanisation catalytique en au moins deux étages (R1, R2) d'un gaz de départ contenant de l'hydrogène et du dioxyde de carbone sous l'influence d'une pression réglable et d'une quantité réglable d'un gaz de dérivation recirculé en amont du premier étage et on contrôle la teneur en méthane du gaz de production, **caractérisé en ce que**
à l'état de taux d'utilisation réduit et compte tenu de la quantité d'écoulement de gaz de départ qui s'y écoule par unité de temps, la teneur en méthane du gaz de production s'élève à plus de 90 % en volume, et **en ce que**, en cas d'augmentation de la quantité de gaz de départ s'écoulant par unité de temps au cours de la transition entre l'état de taux d'utilisation réduit et l'état normal pour garantir cette teneur en méthane dans le gaz de production, on augmente la vitesse de réaction en élevant la pression, et on augmente également la quantité de gaz recirculé en contre-mesure afin d'empêcher la formation concomitante de zones de surchauffe localisées.

2. Procédé selon la revendication 1, dans lequel au moins une partie de l'hydrogène contenu dans le gaz de départ est rendue disponible par consommation d'une charge électrique, notamment au moyen d'une électrolyse, et la quantité de gaz de départ s'écoulant par unité de temps à l'état de taux d'utilisation réduit est fonction de ladite charge consommée.

3. Procédé selon l'une des revendications précédentes, dans lequel la méthanisation se produit sous l'influence d'au moins un autre paramètre appartenant au groupe suivant : la teneur en eau du gaz en amont du premier étage de méthanisation ; la teneur en eau du gaz en amont du deuxième étage de méthanisation ; une quantité d'eau éliminée du mélange de gaz en aval du premier étage de méthanisation ; une puissance thermique servant à chauffer le gaz en amont du premier et/ou du deuxième étage de méthanisation ; une inclusion OUI/NON d'un troisième étage de méthanisation ; la composition du gaz de départ.

4. Procédé selon l'une des revendications précédentes, dans lequel la variation de la pression et/ou de la quantité de gaz recirculé se produit automatiquement et notamment au moyen d'une régulation.

5. Procédé selon l'une des revendications précédentes, dans lequel la variation de la pression et/ou de la quantité de gaz recirculé est soumise à une contrainte supplémentaire à observer.

6. Procédé selon la revendication 5, dans lequel la contrainte supplémentaire est choisie dans le groupe comprenant : la température maximale du catalyseur dans les premier et/ou deuxième étages de méthanisation ; les teneurs minimale et/ou maximale en eau du gaz en amont des premier et/ou deuxième étages de méthanisation ; les quantités minimale et/ou maximale d'eau éliminée en aval de la première étape de méthanisation ; la satisfaction d'un critère concernant la composition du mélange de gaz produit.

7. Procédé selon l'une des revendications précédentes, dans lequel l'observation de la température maximale et, le cas échéant, de la contrainte supplémentaire est commandée par un circuit de régulation associé en conséquence et est notamment contrôlée par ce biais.

8. Procédé selon l'une des revendications précédentes, dans lequel la contre-mesure est entreprise dès qu'un dépassement d'une température prédéfinie est constaté dans le premier étage de méthanisation.

9. Procédé selon la revendication 8, dans lequel une commande d'économie d'énergie est entreprise dès qu'un état stationnaire est atteint relativement à l'écoulement modifié de gaz de départ.

10. Système de réacteur permettant de produire un gaz de production riche en méthane à partir d'un gaz de départ présentant de l'hydrogène et du dioxyde de carbone, comprenant :
au moins deux étages de réacteur (R1, R2) successifs présentant un catalyseur,
un dispositif de contrôle destiné à contrôler la teneur en méthane du gaz de production,
un dispositif de recirculation (RZ) destiné à recycler une partie du gaz, notamment issu du deuxième étage de réacteur, vers une position en amont du premier étage de réacteur,
un dispositif de commande (20) couplé au dispositif de contrôle, et
au moins un dispositif de réglage (7.1, 7.2, 70) commandé par le dispositif de commande (20) et destiné à régler la pression au cours de la méthanisation,
**caractérisé en ce que** le système de réacteur est conçu pour un fonctionnement intermittent entre un état normal et un taux d'utilisation réduit de l'ordre de 60 %, voire moins, du taux d'utilisation maximal et peut être exploité par le biais de la commande du dispositif de commande afin de mettre en œuvre le procédé selon l'une des revendications 1 à 9.

11. Système de réacteur selon la revendication 10, dans lequel un deuxième dispositif de réglage consiste en un dispositif (8.1, 4.1) d'élimination d'au moins une partie de l'eau contenue dans le gaz en aval du premier étage de réacteur.

12. Système de réacteur selon la revendication 11, dans lequel le deuxième dispositif de réglage permet un réglage prédéfinissable du point de rosée.

13. Système de réacteur selon l'une des revendications 10 à 12, dans lequel il est prévu un troisième dispositif de réglage (5) pour apporter de l'eau en amont d'un ou plusieurs des étages de réacteur.

14. Système de réacteur selon l'une des revendications 10 à 13, dans lequel il est prévu un quatrième dispositif de réglage (6) pour chauffer le gaz introduit dans un ou plusieurs étages de réacteur.

15. Système de réacteur selon l'une des revendications 10 à 14, dans lequel le dispositif de recirculation présente un compresseur (70) et/ou un étranglement (90) pilotable.

16. Système de réacteur selon l'une des revendications 10 à 15, présentant en outre un troisième étage de réacteur (R3) activable au moyen du dispositif de commande.

17. Système de réacteur selon l'une des revendications 10 à 16, lequel est susceptible d'être couplé électriquement à un dispositif de production d'hydrogène et y est notamment couplé.

18. Système de réacteur selon l'une des revendications 10 à 17, lequel est susceptible d'être couplé à un dispositif de production de dioxyde de carbone, notamment par l'intermédiaire d'un lavage de gaz, et y est notamment couplé.

19. Système de réacteur selon l'une des revendications 10 à 17, présentant un raccord pour apporter un gaz, notamment un biogaz, utilisable pour former une partie du gaz de départ.

20. Système de réacteur selon l'une des revendications 10 à 19, dans lequel un ou plusieurs des dispositifs de réglage (7.2, 90, 5.1, 8.1, 5.2) sont pilotables par le biais d'une boucle de régulation.
